# EUROPEAN PATENT APPLICATION

(11) **EP 1 956 024 A1**
(43) Date of publication of application: **13.08.2008**
(21) Application number: 07380033.6
(22) Date of filing: 12.02.2007
(51) Int. Cl.: C07F 7/08

(54) **1-Azaspiro[3.5]nonan-2-ona-5,7-carbolact one and 5,7-protected-1-azaspiro[3.5]nonan-2-one derivatives and their use as intermediates in the synthesis of TTX**

(71) Applicant: Laboratorios del Dr. Esteve S.A., 08041 Barcelona (ES)
(72) Inventor: Noheda Marin, Pedro, Instituto de Quimica Orgánica, 28006 Madrid (ES); Tabares Cantero, Nuria, Instituto de Quimica Orgánica, 28006 Madrid (ES); Maroto Quintana, Sergio, Instituto de Quimica Orgánica, 28006 Madrid (ES); Benito Arenas, Raúl, Instituto de Quimica Orgánica, 28006 Madrid (ES); Hojas Garcia, Elena, Instituto de Quimica Orgánica, 28006 Madrid (ES)
(74) Representative: ABG Patentes, S.L.

(57) **Abstract**

The invention provides compounds of formula (I) and methods for their preparation

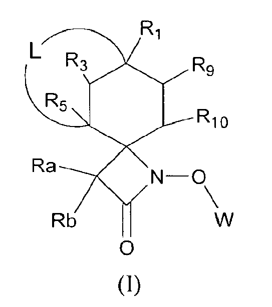

The compounds of the invention are useful intermediates in the synthesis of TTX and analogues thereof.

## Description

### FIELD OF THE INVENTION

The present invention relates to 1-azaspiro[3.5]nonan-2-ona-5,7-carbolactone and 5,7-protected-I-azaspiro[3.5]nonan-2-one derivatives, a method for their synthesis and their use in the synthesis of TTX and TTX analogues.

### BACKGROUND OF THE INVENTION

Tetrodotoxin (TTX), Octahydro-12-(hydroxymethyl)-2-imino-5,9:7,10a-dimethano-10aH-[1,3]dioxocino[6,5-d]pyrimidine-4,7,10,11,12-pentol, is a potent neurotoxin which selectively binds to sodium channel proteins inhibiting its function in the cell membrane. Tetrodotoxin was first isolated in 1909 from the ovaries of the puffer fish, and named after the puffer fish family "Tetraodontidae''. Despite being a small molecule, it has an extremely complex structure characterised by a dioxaadamantane skeleton, a guanidine residue which is part of an hemiaminal, and an orthoacid bridge.

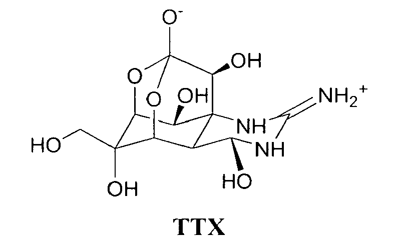

It should also be noted that TTX is usually present as a mixture of two possible tautomers: an orthoester and a hydroxyllactone. The ratio of both tautomers depends on the media in which TTX is present.

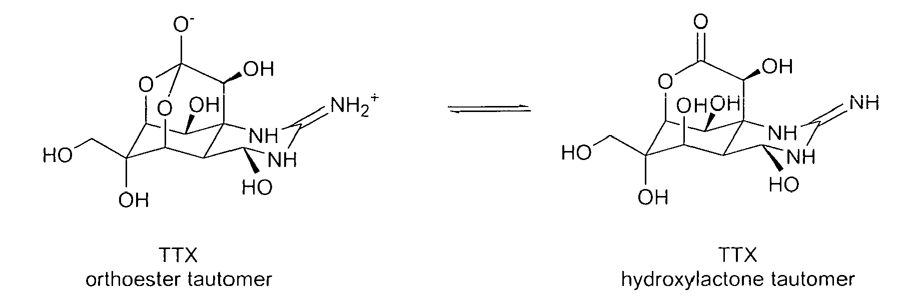

Due to its novel structure and high density of functional groups, only after extensive efforts, Hirata-Goto *et al.* (Goto, T.; Kishi, Y.; Takahashi, S.; Hirata, Y. Tetrahedron 1965, 21, 2059-2088); Tsuda *et al.* (Tsuda, K.; Ikuma, S.; Kawamura, M.; Tachikawa, K.; Sakai, K.; Tamura, C.; Amakasu, O. Chem. Pharm. Bull. 1964, 12, 1357-1374) and Woodward *et al.* (Woodward, R. B. Pure Appl. Chem. 1964, 9, 49-74; Woodward, R. B.; Gougoutas, J. Z. J. Am. Chem. Soc. 1964, 86, 5030) independently arrived at the same structure in 1964. Finally, in 1970 absolute stereochemistry was determined unambiguously by X-ray crystallographic analysis of its derivative (Furusaki, A.; Tomie, Y.; Nitta, I. Bull. Chem. Soc. Jpn. 1970, 43, 3325-3331).

Tetrodotoxin blocks diffusion of sodium through the sodium channel, preventing depolarization and propagation of action potentials in nerve cells. The TTX-Na Channel binding site is extremely tight *(K_{d}* = 10⁻¹⁰ nM). Therefore it is an essential tool in pharmacological studies related to sodium channel proteins.

Further, Tetrodotoxin, due to its analgesic properties, is a promising new drug candidate in the field of pain management

Extraction and purification of natural TTX is complicated, and dependent on the availability of the right animal source. Therefore there is an existing need of providing larger amounts of Tetrodotoxin and its analogues, and researchers seek new cost effective and efficient synthesis. Up to the date, only a handful of total synthesis has been reported (Kishi, Y.; Nakatsubo, F.; Aratani, M.; Goto, T.; Inoue, S.; Kakoi, H.; Tetrahedron Lett. 1970, 59, 5127-5128; Kishi, Y.; Nakatsubo, F.; Aratani, M.; Goto, T.; Inoue, S.; Kakoi, H.; Tanino, H.; Sugiura, S. J. Am. Chem. Soc. 1972, 94, 9217-9219; Kishi, Y.; Nakatsubo, F.; Aratani, M.; Goto, T.; Inoue, S.; Kakoi, H.; Tanino, H.; Sugiura, S. J. Am. Chem. Soc. 1972, 94, 9219-9220; Kishi, Y.; Nakatsubo, F.; Aratani, M.; Goto, T.; Inoue, S.; Kakoi, H.; Tanino, H.; Sugiura, S. J. Am. Chem. Soc. 1972, 94, 9220-9221), Isobe *et al.* in January 2003 (Isobe,M. et al, J.Am.Chem.Soc, 2003, 125, 8798-8805). Dubois *et al.* in June 2003 (Du Bois, J.; Hinman, A. J. Am. Chem. Soc. 2003, 125, 11510-11511), and Isobe *et al.* have reported an additional asymmetric total synthesis comprising 62 steps and an overall yield of approximately 1%, from a vinilic methyl intermediate (Isobe, M.; Urabe, D.; Nishikawa, T. Angew. Chem. Int. Ed. 2004, 43, 4782-4785). Further improvements by the same author have been published on 2006 (Isobe, M.; Urabe, D.; Nishikawa, T.; Urabe, D. Chem. Asian. J. 2006, 1-2, 125-135).

For the purposes of the present invention "TTX analogues" are those described in US 5,846,975 (included herein by reference). From column 3 line 40 to column 6 line 40 US 5,846,975 describes a general formula of known TTX analogues, for example, anhydrotetrodotoxin, tetrodaminotoxin, methoxytetrodotoxin, ethoxytetrodotoxin, deoxytetrodotoxin and tetrodonic acid, 6 epi-tetrodotoxin, 11-deoxytetrodotoxin as well as the hemilactal type TTX derivatives (e.g. *4-epi-TTX, 6-epi-TTX,* 11*-deoxy-*TTX, 4-*epi-*11*-deoxy-*TTX, TTX-8-*O*-hemisuccinate, chiriquitoxin, 11-*nor*-TTX-6(S)-ol, 11-*nor*-TTX-6(R)-ol, 11-*nor*-TTX-6,6-diol, 11-*oxo*-TTX and TTX-11-carboxylic acid), the lactone type TTX derivatives (e.g. 6*-epi-*TTX (lactone), 11-*deoxy*-TTX (lactone), 11-*nor-*TTX-6(S)-ol (lactone), 11-*nor*-TTX-6(R)-ol (lactone), 11-*nor*-TTX-6,6-diol (lactone), 5*-deoxy-*TTX, 5,11*-dideoxy-*TTX, 4*-epi-*5,1 1-*didroxy*-TTX, 1-*hydroxy*-5,11-*dideoxy*-TTX, 5,6,11-*trideoxy*-TTX and 4-*epi*-5,6,11-*trideoxy*-TTX) and the 4,9-anhydro type TTX analogs (e.g. 4,9*-anhydro-*TTX, 4,9*-anhydro-*6*-epi-*TTX, 4,9-*anhydro*-11- *deoxy*-TTX, 4,9-*anhydro*-TTX-8-*O*-hemisuccinate, 4,9-*anhydro*-TTX-11-*O*-hemisuccinate). The typical analogues of TTX possess only 1/8 to 1/40 of the toxicity of TTX in mice, based upon bioassay in mice. It has been observed that these derivatives produce joint action, and do not interact adversely.

Also, Isobe has reported the synthesis of different unnatural analogues of Tetrodotoxin following similar strategies as those reported for Tetrodotoxin. Concretely, Isobe *et al.* has reported the synthesis of (-)-5,11-Dideoxytetrodotoxin (Isobe, M.; Asai, M.; Ohyabu, N.; Yamamoto, N.; Nishikawa, T. Angew. Chem. Int. Ed. 1999, 38, 3081-3084), (-)-8,11-Dideoxytetrodotoxin (Isobe, M.; Asai, M.; Iwabuchi, T.; Yoshida, K.; Urabe, D.; Nishikawa, T. Org. Lett. 2002, 16, 2679-2682; Isobe, M.; Asai, M.; Iwabuchi, T.; Yoshida, K.; Urabe, D.; Nishikawa, T. Chem. Eur. J. 2004, 10, 452-462) and 11-Deoxytetrodotoxin (Isobe, M.; Asai, Nishikawa, T. J. Am. Chem. Soc. 2002, 124, 7847-7852).

Finally. Sato *et al.* have recently reported an asymmetric total synthesis of Tetrodotoxin from myo-inositol in approximately 0.14% overall yield after 40 steps (Sato, K.; Akai, S.; Sugita, N. ; Ohsawa, T. ; Kogure, T. ; Shoji, H. ; Yoshimura, J. J. Org. Chem. 2005, 70, 7496-7504).

Also, further discussions regarding different synthetic approaches to Tetrodotoxin and its analogues may be reviewed in Koert, U. T. Angew. Chem. Int. Ed. 2004, 43, 5572-55769.

In view of all of the above there is an existing need of providing an alternative cost effective and efficient synthesis of Tetradotoxin and analogues thereof, which can be used for the industrial production of sufficient amounts of these compounds. Further new analogues with useful biological and pharmacological properties will be readily available by means of new synthetic strategies.

### BRIEF DESCRIPTION OF THE FIGURES

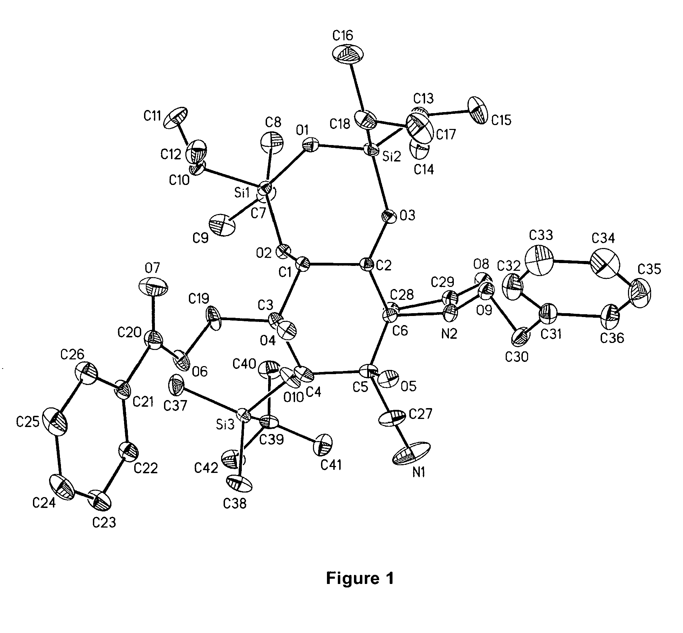

**Figure 1** is a representation of the molecular structure obtained using X-rays diffraction of *rac-*(4*R*,5*R*,6*R*,7*S*,8*S*,9*S*)-1-benzyloxy-7-(benzoyloxymethyl)-6-(tert-butyldimethyl silyloxy)-5-cyano-5,7,8,9-tetrahydroxy-8,9-*O*-(1,1,3,3-tetraisopropyldisiloxane-1,3-diyl)-1-azaspiro[3.5]nonan-2-one (7). Hydrogen atoms have been omitted for the sake of clarity.

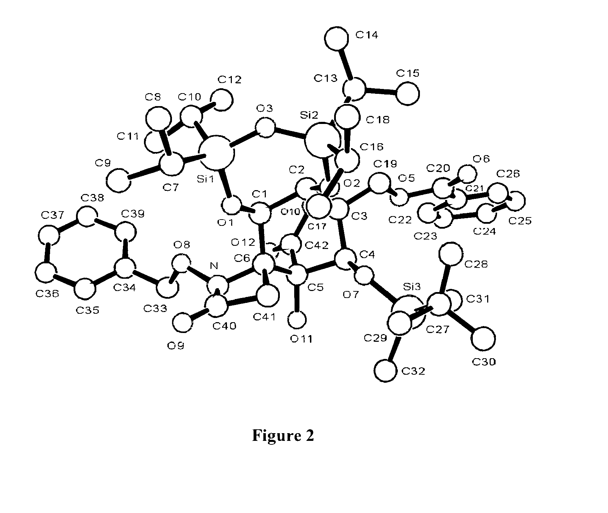

Figure 2 is a representation of the molecular structure obtained using X-rays diffraction of *rac-*(4*S,*5*R,*6*S,*7*R,*8*S,*9*S)-*1-benzyloxy-7-(benzyloxylmetyl)-6-(*tert-*butyldimethylsililoxi)-5,8,9-trihydroxy-8,9-*O*-(1,1,3,3-tetraisopropyldisiloxane-1,3-diyl)-1-azaspiro[3.5]non-2-ona-5,7-carbolactone **(10)**. Hydrogen atoms have been omitted for the sake of clarity.

### SUMMARY OF THE INVENTION

In their ongoing efforts the inventors have found that a new class of 1-azaspiro[3.5]nonan-2-ona-5,7-carbolactone and 5,7-protected-1-azaspiro[3.5]nonan-2-one derivatives are promising intermediates for the synthesis of TTX and analogues thereof. Thus, a first aspect of the invention is directed to a compound of formula (I) as defined below (from now on, "compound of formula (I)" or "compound of the invention").

A second aspect of the invention is a method for the synthesis of a compound of formula (I) from a compound of formula (II), (III), (IV) or (V) as defined below.

A third aspect of the invention is directed to the use of a compound of formula (I) as intermediate in the synthesis of TTX or analogues thereof.

### DETAILED DESCRIPTION OF THE INVENTION

For the purposes of the present invention the compounds of general formula (I), (Ia), (la'), (Ib) and (Ib') have been also nominated as "compounds of the invention". Compounds described herein ((I), (Ia), (Ia'), (Ib), (Ib'), (II), (III), (IV), (V), (VI), (VII), (VIII)) have been numerated as described below when referred to:
- Compounds of formula (II), (III), (IV), (V), (VI), (VII) and (VIII): 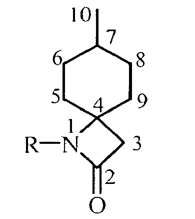
- Compounds of formula (Ia) and (Ia'): 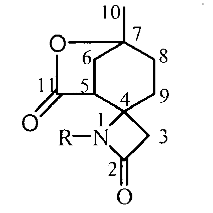
- Compounds of formula (Ib) and (Ib'):

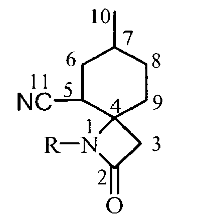

However, IUPAC prelation rules have been applied when naming individual compounds (see examples).

In the present application the following numbering will be used when reference is made to TTX or analogues thereof (see figure 1, page 7497 in Sato, K.; Akai, S.; Sugita. N.: Ohsawa, T.: Kogure. T.: Shoji, H.: Yoshimura, J. J. Org. Chem. 2005. 70, 7496-7504):

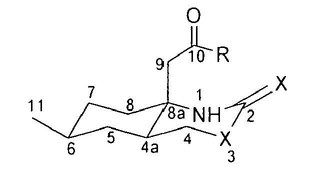

### Compounds of formula (I)

As mentioned above, a first aspect of the present invention is directed to a compound of formula (I) 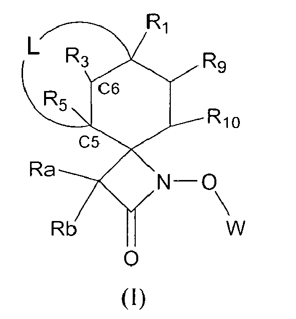 wherein
R₁ is selected from hydrogen, cyano, substituted or unsubstituted alkyl, alkyl-OPr, substituted or unsubstituted alkenyl, substituted or unsubstituted alkynyl and NReRf, wherein Re and Rf are each independently selected from H, OH, OPr, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy, substituted or unsubstituted amino and halogen; and
R₃ is selected from hydrogen, OH, OPr or =O; or
R₁ and R₃ together form a group selected from -CH₂-O-Pr-O- and -CH₂-O-PrC(R₁₁R₁₂)-O-, the oxygen terminus being attached to the C6 carbon atom (as shown in the above figure, C6 is the carbon atom which supports R₃); wherein R₁₁ is selected from the group consisting of substituted or unsubstituted alkyl, substituted or unsubstituted cycloalkyl, substituted or unsubstituted alkenyl, substituted or unsubstituted aryl, substituted or unsubstituted aralkyl, substituted or unsubstituted alkoxy, substituted or unsubstituted aryloxy; and R₁₂ is selected from the group consisting of cyano, substituted or unsubstituted alkyl and aziridine;
R₉ and R₁₀ are each independently selected from hydrogen, OH, OPr or =O;
R₅ is selected from the group consisting of hydrogen, -CN,-OH, OPr and a carbonyl derivative of formula -C(=O)Rg, wherein Rg is a selected form the group consisting of hydrogen, substituted or unsubstituted alkoxy, substituted or unsubstituted aryloxy and substituted or unsubstituted amine;
L is selected from the group consisting of
   - -O-C(=X)-, wherein X is selected from NPrn, NH or O, and the carbon atom supporting X is attached to C5 (as shown in the above figure, C5 is the carbon atom which supports R5);; and
   - Z, wherein Z is selected from the group consisting of -O-C(R₆R₇)-O-, - O-Si(R₆R₇)-O- and -O-Si(R₆R₇)-O-Si(R₆R₇)-O-, wherein each of R₆ and R₇ are independently selected from the group consisting of hydrogen, substituted or unsubstituted alkyl, substituted or unsubstituted cycloalkyl, substituted or unsubstituted alkenyl, substituted or unsubstituted alkynyl, substituted or unsubstituted aryl and substituted or unsubstituted heterocyclyl;
W is selected from the group consisting of-H, substituted or unsubstituted alkyl, substituted or unsubstituted aryl, substituted or unsubstituted aralkyl, substituted or unsubstituted heterocyclyl and substituted or unsubstituted alkenyl;
Ra and Rb are each independently selected from H, OH, OPr, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy, substituted or unsubstituted amino and halogen;
Pr is a hydroxyl protecting group; and
Pm is an amino protecting group;
or its tautomers, salts or solvates thereof.

The compounds of formula (I) are specially interesting intermediates for the synthesis of TTX and analogues thereof because unwanted dehydration, elimination and epimerization reactions are avoided. Due to the dense functionalitation of the compounds of the invention, these side reactions are frequent. The presence of acid hydrogen atoms in β position with respect to heteroatoms or potential leaving groups may give raise to elimination reactions. Also, epimerization and substitution processes facilitated by anchimeric assistance are a problem. Anchimeric assistance is also known as neighbouring group participation or NGP and has been defined by IUPAC as the interaction of a reaction centre with a lone pair of electrons in an atom or the electrons present in a sigma bond or pi bond. For example, if a heteroatom with a lone pair of electrons is in alfa position with respect to a potential leaving group, said lone pair of electrons may displace the leaving group, activating the carbon atom which supported said leaving group. However, the carbon atom which supports the heteroatom also becomes activated, which is a source of unwanted substitution reactions, lost of regioselectivity or epimerization processes. For example, if two vecinal axial hydroxyl groups in a cyclohexane are in relative anti positions, dehydration may readily proceed. This is the situation for many intermediates of TTX, specially with regard to positions C5, C6, C7 and/or C8. Fixing cyclation between the substituents of C5 and C7 prevents such dehydrations.

The invention also provides salts of compounds of formula (I) with biologically and pharmacologically acceptable inorganic and organic acids, non-limiting examples of which are sulphates; hydrohalide salts; phosphates; lower alkane sulphonates; arylsulphonates; salts of C₁ -C₂₀ aliphatic mono-, di- or tribasic acids which may contain one or more double bonds, an aryl nucleus or other functional groups such as hydroxy, amino, or keto; salts of aromatic acids in which the aromatic nuclei may or may not be substituted with groups such as hydroxyl, lower alkoxyl, amino, mono- or di- lower alkylamino sulphonamido. Also included within the scope of the invention are quaternary salts of the tertiary nitrogen atom with lower alkyl halides or sulphates, and oxygenated derivatives of the tertiary nitrogen atom, such as the N-oxides. In preparing dosage formulations, those skilled in the art will select the pharmaceutically acceptable salts.

The term "solvate" according to this invention is to be understood as meaning any form of the active compound according to the invention which has another molecule (most likely a polar solvent) attached to it via non-covalent bonding. Examples of solvates include hydrates and alcoholates, e.g. methanolate.

Tautomers of the compounds described herein are also included in the scope of the present application. For example, tautomers of the compounds of formula (I), (Ia), (Ia'), (Ib), (Ib'), (II), (III), (IV), (V), (VI), (VII) or (VIII) may arise from the rearrangement of the azaspiro[3.5]none to an azaoxaspiro[4.5]decenone:

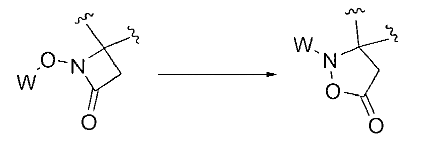

This tautomerization may take place due to strong acid or basic condition as described in our co-pending application EP06380204.5, as well as a consequence of heat (thermal rearangement). These tautomers are also suitable intermediates for the synthesis of TTX and analogues thereof and are included within the scope of the invention.

Another example of tautomerism of the compounds of formula (I), (Ia), (Ia'), (Ib), (Ib'), (II), (III), (IV), (V), (VI), (VII) or (VIII) are enolates of carbonyl compounds. The acid hydrogens which are alfa to a carbonyl functionality may be readily displaced to form the corresponding enol-tautomer, which is usually far less stable than the corresponding carbonyl-tautomer. However, the enol may become more stable or trapped. Thus, the enol tautomers are also included in the scope of the invention. This is specially relevant in the case of alfa-hydroxyketones, wherein the two carbon atoms may share the carbonyl/enol tautomeric character in variable degrees:

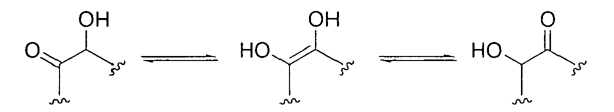

These tautomers are also included within the scope of the invention.

According to an embodiment of the present invention the compound of formula (I) is a compound of formula (Ia) 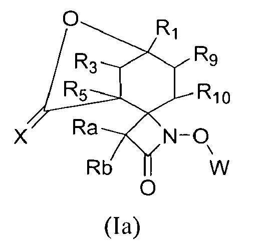 wherein
R₁, R₃, R₉, R₁₀, Ra, Rb, W and X are as defined in formula (I): and
R₅ is selected from the group consisting of hydrogen, -OH or OPr ; or its tautomers, salts or solvates thereof.

According to a further embodiment of the present invention the compound of formula (I) is a compound of formula (Ib) 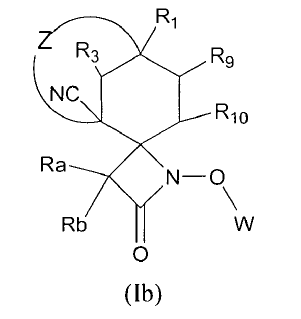 wherein
R₁, R₃, R₉, R₁₀, Ra, Rb, Z and W are as defined in formula (I); or its tautomers, salts or solvates thereof.

According to a preferred embodiment, R₁ is -CH₂-OPr, preferably selected from the group consisting of-CH₂-OC(=O)alkyl and -CH₂-OC(=O)Aryl.

According to a preferred embodiment, R₁ and R₃ together are -CH₂-O-Pr-O-, wherein Pr is a hydroxyl protecting group.

According to a preferred embodiment, R₉ and R₁₀ together are -O-Pr-O-, wherein Pr is a hydroxyl protecting group.

According to a preferred embodiment, a compound of formula (Ia) is a compound of formula (Ia') 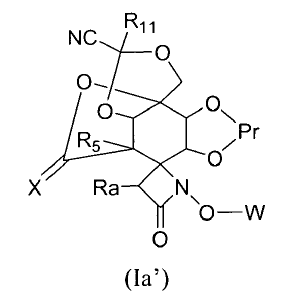 wherein R₅, R₁₁, Ra, X, Pr and W are defined as in formula (Ia); or its tautomers, salts or solvates thereof.

According to a preferred embodiment, R₅ is hydrogen.

According to a preferred embodiment. R₅ is -OH.

According to a preferred embodiment, X is O.

According to a preferred embodiment, a compound of formula (Ib) is a compound of formula (Ib⁻) 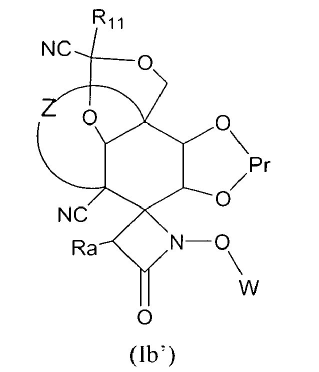 wherein Ra, R₁₁,, Z, Pr and W are defined as in formula (I); or its tautomers, salts or solvates thereof.

According to a preferred embodiment, when R₁ and R₃ together are -CH₂-O-Pr-O-.

According to a preferred embodiment R₁ and R₃ together are -CH₂-O-C(R₁₁R₁₂)-O-.

According to a preferred embodiment R₁₁ is substituted or unsubstituted aryl.

According to a preferred embodiment R₁₂ is cyanide.

According to a preferred embodiment, W is aralkyl, preferably -CRcRd-aryl, wherein Rc and Rd are each independently selected from H, OH, OPr, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy, substituted or unsubstituted amino and halogen.

According to a preferred embodiment, Rc and Rd are different.

According to a preferred embodiment, W is -CRcH-aryl, wherein Rc is not hydrogen, that is, wherein Rc is selected from the group consisting of OH, OPr, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy, substituted or unsubstituted amino and halogen.

According to a preferred embodiment, Rc and Rd are both hydrogen.

According to a preferred embodiment, Ra and Rb are different. According to a preferred embodiment, Ra and Rb are both hydrogen.

### Synthesis of the compounds of formula (I)

There are different synthetic approaches for the preparation of compounds of formula (I):
(i) cyclizating a cis-compound of formula (II);
(ii) reacting a compound of formula (III) with ⁻CN and, optionally, if the cyano group and the hydroxyl group are not in cis relative position, epimerizating the resulting compound of formula (II);
(iii) dihydroxylating a compound of formula (IV);
(iv) hydrogenating a compound of formula (V); or
(v) protecting the diol on the C5 and C7 positions of a trans-compound of formula (II). A trans-compound of formula (II) is a compound of formula (II) wherein the cyanide C5 is cis with regard to R₂.

The above mentioned procedures are explained in detail below. First, the methods for the synthesis of compounds of formula (Ia) are explained (methods (i), (ii), (iii) and (iv)) and then method (v) for the synthesis of compounds of formula (Ib).

### Synthesis of the compounds of formula (Ia)

The immediate precursors of the compounds of formula (Ia) are those wherein a hydroxyl group in C7 and a cyano group in C5 are in cis relative position. The inventors have found that in such compounds cyclization takes place readily to yield the compounds of formula (Ia).

A first method (method (i)) for the synthesis of a compound of formula (I), comprises the cyclation between the R₂ group and the cyano group of a compound of formula (II) 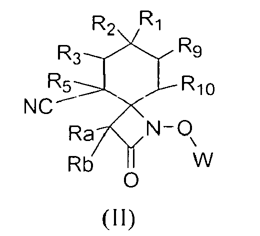 wherein
R₁, R₃, R₉, R₁₀, Ra, Rb and W are as defined in formula (I);
R₅ is selected from the group consisting of hydrogen, -OH and OPr; and
R₂ is -OH or -OPr, Pr being a hydroxyl protecting group; wherein the cyano group and R₂ are in relative cis positions;
or its tautomers, salts or solvates thereof.

Cyclation takes place easily under acid or basic conditions to yield a compound of formula (Ia).

If R₂ is -OPr deprotection liberates the hydroxyl group necessary for cyclation, which may proceed without further purification of the intermediate in a one-pot reaction.

Methods for obtaining the compounds of formula (II) are described in the PCT patent application PCT/EP2006/068236 (not published) in the name of the same applicant. Thus, for example, PCT/EP2006/068236 already discloses that a compound of formula (II) may be obtained by the nucleophylic attack of a cyanide anion to the carbonyl moiety of a compound of formula (III) 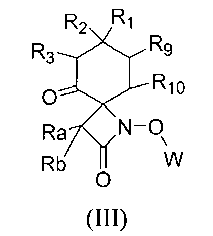 wherein R₁, R₃, R₉, R₁₀, Ra, Rb and W are as defined in formula (I); and R₂ is - OH or -OPr, Pr being a hydroxyl protecting group; or its tautomers, salts or solvates thereof.

The cyanide anion necessary for the reaction may be obtained from various sources known to the skilled person. For example, such sources may be selected from the following reagent systems: Ph₃P/diethyl azodicarboxylate (DEAD)/alcohol/acetone cianhydrin *(*Synth. Com. 1995, 25, 1545-1550; Synth. Com. 1993, 23, 2481-2484).

Other sources of cyanide anion are based on reagent systems comprising NaCN. Conditions already described in the art comprise a mixture of NaCN with one of the following reaction systems: DMSO or nBu₄NCN/DMF/heat *(*J. Med. Chem. 1991, 56, 3009-3016; J. Med. Chem. 1966, 31, 2933-2941), DMSO/heat *(*J. Med. Chem. 2001, 44, 94-104; Tetrahedron Asymmetry, 1996, 7, 1967-1972), Bu₄NCN/DMSO/heat (Bioorg. Med. Chem. Lett. 1999, 9, 841-846), DMF/heat *(*Tetrahedron Letters, 1998, 39, 3357-3358; J. Med. Chem. 1994, 37, 4195-4210), [(CH₃)₂N]₃PO *(*J. Chem. Soc. Chem. Com. 1982, 7, 404-406) or conditions wherein sodium bicarbonate is present, such as NaCN/NaHCO₃/H₂O/Et₂O/r.t., NaCN/NaHCO₃/Et₂O/r.t. or NaCN/NaHCO₃/THF/-H₂O *(*J. Med. Chem. 1992, 35, 2721-2727; Carbohydrate Resarch 1991, 216, 399-411; J. Med. Chem. 1998, 41, 4636-4647 ; Carbohydrate Resarch 1991, 216, 399-411). Also, NaCN in the presence of 1-Methyl-1H-pyrrole provides the desired cyanide anion.

In another embodiment, the reaction may proceed in the presence of KCN (J. Am. Chem. Soc. 1958, 80, 4677-4680). Conditions already described in the art comprise a mixture of KCN with one of the following reaction systems: Et₂O/H₂O *(*Tetrahedron Letters, 2001, 42, 6259-6262), THF/r.t. *(*Carbohydrate Resarch 1994, 254, 133-140) or CH₂Cl₂/H₂O/r.t., with or without NaHCO₃ (J. Chem. Soc. Perkin Trans 1. 1985, 1067-1072).

In another embodiment, the reaction may proceed in the presence of LiCN. Conditions already described in the art comprise a mixture of LiCN with one of the following reaction systems: Me₃SiCl/THF/r.t. *(*Synthesis, 1986, 12, 1054-1055), DMF/[(CH₃)₂N]₃PO *(*Bioorg. Med. Chem. Lett. 1996, 6, 1897-1900), diethyl cyanidophosphate/THF/r.t. *(*Chem. Pharm. Bull. 1986, 34, 4620-4628; Tetrahedron Letters, 1989, 30, 3681-3684*)* or DMF *(*Bioorg. Med. Chem. Lett. 2000, 10, 2417-2419).

Copper cyanide is also a useful source of cyanide anion (CuCN/HNaCO3/H₂O/EtO₂ ; Tetrahedron. 1988, 44, 4895-4904).

In another embodiment, the reaction may proceed in the presence of HCN. Conditions already described in the art comprise a mixture of HCN with one of the following reaction systems: Pyridine/r.t. (J. Chem. Soc. Perkin Trans 1. 1994, 1067-1072), Et₃N/CH₂Cl₂/0°C *(*Carbohydrate Resarch 1986, 155, 236-246) or in situ generation of HCN by mixing Zₙ(CN)₂ and AlCl₃ *(*Tetrahedron Asymmetry. 1990. 1, 187-198).

Thus, according to one embodiment, the reagent which provides a cyanide anion has a formula MCN, wherein M is lithium, potassium, sodium or copper.

Trialkylsylil cyanides are also wide spread sources of cyanide anions *(*J. Orig. Chem. 2003, 3094-3103; J. Am. Chem. Soc. 1973, 5822-5823; J. Chem. Soc. Perkin Trans 1. 1988, 2305-2307). Conditions already described in the art comprise a mixture of trialkylsilyl cyanide with one of the following reaction systems: TMSCN/DABCO or TMSCN/(DHQ)₂AQN *(*J. Am. Chem. Soc. 2003, 125, 9900-9901), TMSCN/BF₃/CH₂Cl₂/r.t. or TMSCN/BF₃·Ei₂O/CH₂Cl₂/r.t. *(*Tetrahedron Letters, 1990, 31, 71-74; Tetrahedron Letters, 1990, 31, 71-74; Tetrahedron Letters, 1990, 31, 71-74; Carbohydrate Resarch 1994, 258, 77-86), TMSCN/crown ether/KCN(cat)/toluene/-30°C (J. Am. Chem. Soc. 1985, 107, 4577-4579; Tetrahedron Letters, 1994, 35, 7901-7904), TMSCN/amine/CH₂Cl₂/0°C *(*Chemistry Letters. 1991, 125, 537-540), TMSCN/I₂/20-30°C *(*Tetrahedron 2000, 35, 6533-6540), TMSCN/ZnI₂/heat *(*Tetrahedron Asimmetry. 1994, 9, 805-816), TMSCN/CaO/Toluene/-40°C (J. Chem. Soc. Chem. Com. 1991, 15, 1035-1036), TMSCN/Ph₃P(cat)/CH₂Cl₂ *(*Tetrahedron Letters, 1986, 27, 2757-2760), TMSCN/Lewis acid *(*Tetrahedron Letters, 1983, 39, 967-974), TMSCN/catalyst *(*Tetrahedron 2001, 57, 771-779); TMSCN/ZnI₂(cat) *(*Tetrahedron 1994, 50, 2821-2830), TBDMSCN/ZnI₂/CH₂Cl₂ *(*J. Org. Chem. 1993, 58, 159-164; Bull. Chem. Soc. Jpn. 2001, 74, 997-1008), (alkyl)₃SiCN/LiCl(cat)/no solvent *(*J. Org. Chem. 2005, 70, 6530-6532).

According to a further embodiment, reagents which provide a cyanide anion are a dialkylaluminium cyandes of formula (alkyl)₂-Al-CN or a trialkylsilyl cyanide of formula (alkyl)₃Si-CN, which are commercially available.

According to a preferred embodiment, the compound of formula (III) reacts in the presence of TMSCN or Et₂AlCN.

If R₂ in the compound of formula (III) is a deprotected hydroxyl group or is deprotected due to reaction conditions, the attack of the cyanide anion to the carbonyl group in C5 forms a compound of formula (II) which may undergo further cyclation in the same reaction media or during workup, to yield a compound of formula (Ia).

The attack of the cyanide anion to a compound of formula (III) may yield two stereoisomers of formula (II), one wherein the cyanide C5 is cis with regard to R₂ (cis-compound of formula (II)), as a result of an equatorial attack, and one wherein it is trans (trans-compound of formula (II)), as a result of an axial attack. As mentioned before, cis-compounds of formula (II) yield compounds of formula (Ia), while trans-compounds of formula (II) do not. The proportion cis:trans is variable and it can be controlled by an appropriate selection of reagents and conditions.

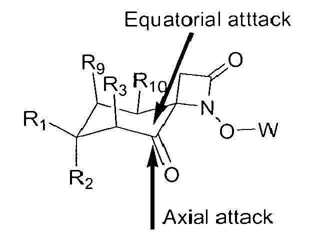

For example, the reaction of a compound of formula (III) with Et₂AlCN or NC-C(=O)-CH₃ usually provides a trans-compound of formula (II), as a result of an equatorial attack, while the reaction in the presence of TMSCN provides a cis-compound of formula (II), as a result of an axial attack.

Regardless of the stereochemistry of the reaction, it is also possible to transform any trans-compound of formula (II) into a cis-compound of formula (II) by a process of epimerization. For example, the reaction between a trans-compound of formula (II) and di-*tert*-butylsilyl bis(trifluomethane)sulfonate in the presence of triethylamine yields the desired cis-compound of formula (II) :

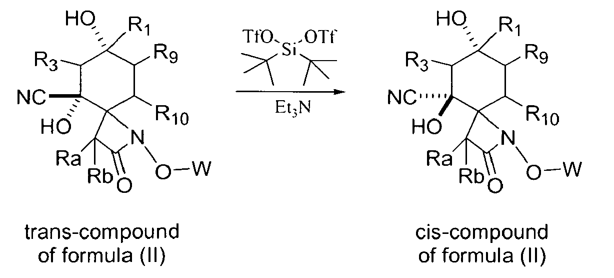

As mentioned above, this cis-compound of formula (II) is readily transformed into a compound of formula (I), either by further treatment or in the same reaction media:

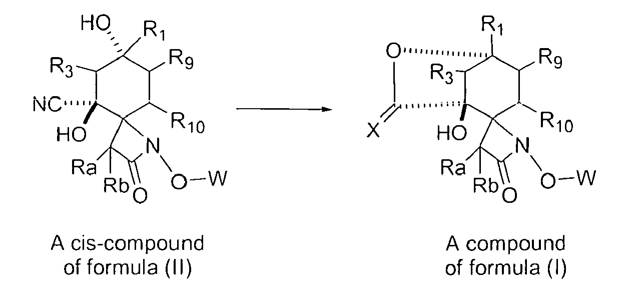

It is also possible to epimirize a trans-compound of formula (II) into a cis-compound of formula (II) under conditions such as those described on page 7499 of Sato, et al, J. Org. Chem., 2005, 70, 7496-7504 (see discussion regarding the transformation of the compound 26a into the compound 26)

Thus, a second method (method (ii)) for the synthesis of a compound of formula (1), comprises the following steps:
a) reacting a compound of formula (III) as defined above in the presence of a reagent which provides cyanide anion (⁻CN); and, if necessary,
b) epimerizing the C5 carbon atom of the compound of formula (II)obtained.

Formation of compounds of formula (III) has been already disclosed in the PCT patent application PCT/EP2005/005149 in the name of the applicant. For example, the compound of formula (III) may be obtained by oxidation of a compound of formula (VI) 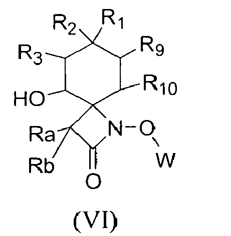 wherein R₁, R₂, R₃, R₉, R₁₀, Ra, Rb and W are as defined in formula (III); or its tautomers, salts or solvates thereof.

According to a preferred embodiment, the compound of formula (VI) is a compound of formula (VII) 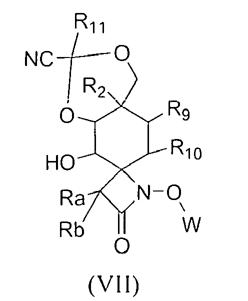 wherein R₂, R₉, R₁₀, R₁₁, Ra, Rb and W are as defined in formula (I); or its tautomers, salts or solvates thereof. Oxidation of said compound of formula (VII) provides a compound of formula (III), which may be transformed into a compound of formula (II) by the methods described above.

The compounds of formula (VII) can be prepared by reaction of a compound of formula (VIII) 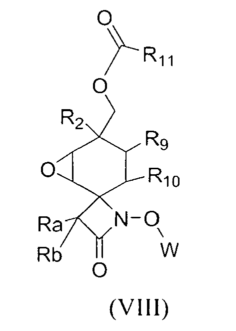 wherein R₂, R₉, R₁₀, R₁₁, Ra, Rb and W are as defined in formula (I); or its tautomers, salts or solvates thereof; in the presence of a reagent which provides a cyanide anion.

Therefore, according to a preferred embodiment, the method of the invention comprises
a) reacting a compound of formula (VIII), as defined above, in the presence of a reagent which provides a cyanide anion, to obtain a compound of formula (VII) as defined above;
b) oxidizing said compound of formula (VII) to obtain a compound of formula (III); and
c) reacting the compound of formula (III) obtained in step b) in the presence of a reagent which provides cyanide anion (⁻CN); and, if necessary, epimerizing the C5 carbon atom of the compound of formula (II) obtained.

In the above reaction, reagents which provide a cyanide anion are preferably the same as in steps a) and c). Also, dialkylaluminium cyandes of formula (alkyl)₂-Al-CN are preferred.
The use of the same reagent in both reactions makes the overall process more simple, which is especially important on the industrial scale.

The compounds of formula (VIII) can be prepared by methods already disclosed in PCT/EP2005/005149 and PCT/EP2006/068236.

As mentioned above, cyclation between the cyanide group of C5 and the hydroxyl group of C7 takes place readily. Since such cyclation may take place in the reaction media once both groups are cis, without the need of separating the intermediate compound of formula (II), the inventors have further devised methods (iii) and (iv) for the synthesis of the compounds of formula (Ia).

Method (iii) comprises dihydroxylating the double bond between C7 and C10 of a compound of formula (IV) 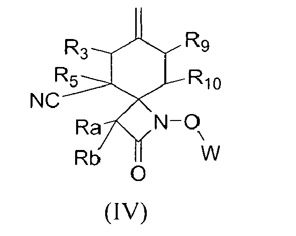 wherein
R₃, R₉, R₁₀, Ra, Rb and W are as defined in formula (I); and
R₅ is selected from the group consisting of hydrogen, -OH and OPr; or its tautomers, salts or solvates thereof.

That is, the hydroxyl group on C7 is introduced in a compound wherein the cyano group on C5 is already present

Dihydroxylation may be performed by methods described in PCT/EP2005/005149 (see examples 15 and 22 therein).

Method (iv), comprises hydrogenation of a compound of formula (V): 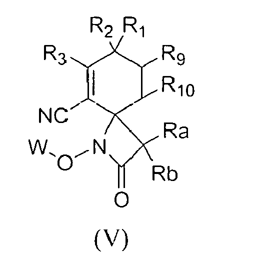 wherein
R₁, R₃, R₉, R₁₀, Ra, Rb and W are as defined in formula (I), and
R₂ is -OH or -OPr;
or its tautomers, salts or solvates thereof, to yield a compound of formula (Ia).

That is, according to method (iv), both, the hydroxyl group on C7 and the cyano group on C5 are present in the starting material (compound of formula (V)), but not in the required cis position. Effecting a reaction which provides the desired cis position provides the desired compounds of formula (II) with the necessary stereochemistry for cyclation.

Preparation of the compounds of formula (V) has been already described in PCT/EP2006/068236. For example, they may be prepared from compounds of formula (III)

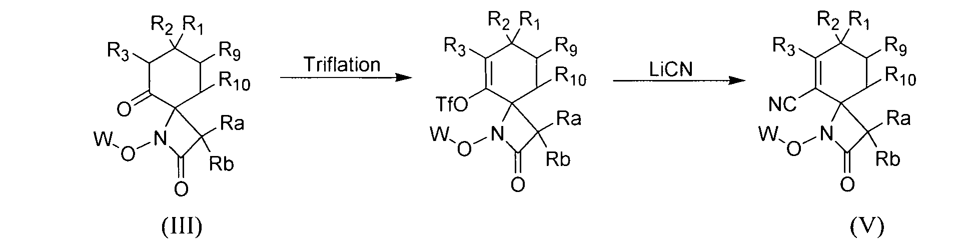

The reaction conditions for the above mentioned reactions are known to the skilled person. Triflation is a standard reaction which may be performed, for example, following conditions described in Tetrahedron Lett. 1984, 25, 595-598. Introduction of a cyano group may be performed by nucleophilic substitution following, for example, the conditions described in Can. J. Chem. 1993, 71, 1867-1872.

### Synthesis of compounds of formula (Ib)

Contrary to the compounds of formula (Ia), the immediate precursors of the compounds of formula (Ib) are the trans-compounds of formula (II), wherein the hydroxyl groups in C5 and C7 have the appropriate cis configuration:

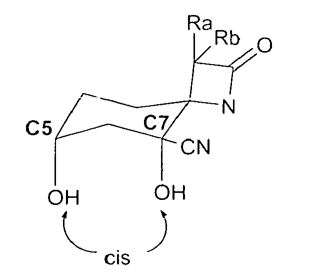

Once the C5 and C7 hydroxyl groups are in cis position, it is possible to simultaneously protect both with a diol protecting group by methods known in the art. Methods for protecting 1,3-diols can be found in the literature (see Greene and Wuts "Protective Groups in Organic Synthesis", John Wiley & Sons, Inc., New York, 1999). Non limitative examples of reagents for diol protection which may provide the compounds of formula (Ib) from the trans-compounds of formula (II) are listed (typical non-limitative are indicated in the parenthesis):
Cyclic acetals and ketals of formula (alkylO)₂C(R₆R₇) (e.g. (MeO)₂CH₂/LiBr/TsOH/CH₂CL₂/r.t.), acetonides of formula (alkylO)C(R₆)=CH₂ (e.g. CH₃C(OMe)=CH₂/TsOH/DMF), benzylidene acetals of formula ArCHO or aralkyl-CHO (e.g. PhCHO/ZnCl₂, PhCHO/DMSO/H₂SO₄, PhCHO/TsOH/reflux/-H₂O, PhCHBr₂/Pyr, PhCH(OMe)₂/HBF₄/Et₂O/DMF, PhCH(OMe)₂/SnCl₂/DME/heat, Ph(OCH₂CH₂CH=CH₂)₂ /CSA/NBS, KHMDS/PhCHO), methoxybenzyllidene acetals (*p*-MeOC₆H₄CHO/acid, p-MeOPhCHO/TMSOTf/CH₂Cl₂/-78° C, *p-*MeOC₆H₄CH₂OMe/DDQ/CH₂Cl₂, - *p*-MeOC₆H₄CH(OMe)₂/acid(CSA)/DMF, *p-*MeOC₆H₄CH₂OMe/ZnCl₂), di-*t*-butylsilylene group of formula (*t*-Bu)₂Si(OR)₂ ((*t-*Bu)₂SiCl₂/CH₃CN/TEA/HOBt, (*t*-Bu)₂Si(OTf)₂/2,6-lutidine/CHCl₃, (*t-*Bu)₂SiCl₂/AgNO₃/Pyr/DMF, (*t*-Bu)₂Si(OTf)₂/DMF/Pyr), 1,3-(1,1,3,3-Tetraisopropyldisiloxanylidene) derivates of formula TIPDS(OR)₂ (TIPDSCl₂/DMF/Imidazole, TIPDSCl₂/Pyr, TIPDSCl₂/AgOTf/sym-collidine/DMF) or 1,1,3,3- Tetra-t-butoxydisiloxanylidene derivates of formula TBDS(OR)₂ (1,3 Dichloro-1,1,3,3-tetra-t-butoxydisiloxane/Pyr) or compounds of formula 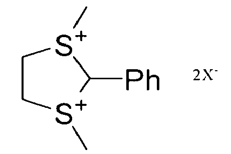 wherein X is FSO₃ or BF₄ (K₂CO₃ or Pry/CH₂Cl₂)

Although each of the procedures described herein use known reagents, their application in the present invention provides new compounds and unexpected results in terms of reactivity and selectivity.

In view of the above, a second aspect of the invention is a method for the synthesis of a compound of formula (I) which comprises one of the following methods:
(i) cyclation between the R₂ group and the cyano group of a compound of formula (II) 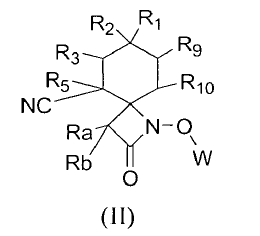 wherein
   R₁, R₃, R₅, R₉, R₁₀, Ra, Rb and W are as defined in formula (I); and
   R₂ is -OH or -OPr, Pr being a hydroxyl protecting group; wherein the cyano group and R₂ are in relative cis positions;
   or its tautomers, salts or solvates thereof;
(ii) reacting a compound of formula (III) 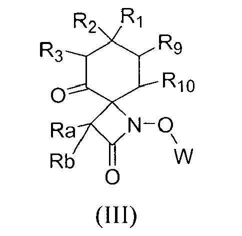 wherein
   R₁, R₃, R₉, R₁₀, Ra, Rb and W are as defined in formula (1); and
   R₂ is -OH or -OPr, Pr being a hydroxyl protecting group;
   or its tautomers, salts or solvates thereof;
   in the presence of a reagent which provides cyanide anion (⁻CN); and, if necessary, epimerizing the C5 carbon atom of the compound of formula (II) obtained;
(iii) dihydroxylating the double bond between C7 and C10 of a compound of formula (IV) 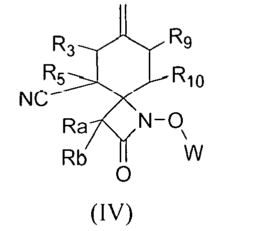 wherein
   R₃, R₅, R₉, R₁₀, Ra, Rb and W are as defined in formula (I);
   or its tautomers, salts or solvates thereof;
(iv) hydrogenate a compound of formula (V) 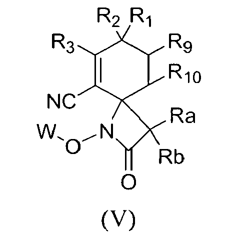 wherein
   R₁, R₃, R₉, R₁₀, Ra, Rb and W are as defined in formula (I); and
   R₂ is -OH or -OPr;
   or its tautomers, salts or solvates thereof; or
(v) diol protection of hydroxyl groups on C5 and C7 of a compound of formula (II) 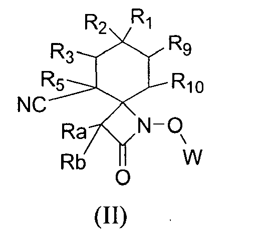 wherein
   R₁, R₃, R₉, R₁₀, Ra, Rb and W are as defined in formula (I); and
   R₂ and R₅ are both independently a -OH group in cis relative positions;
   or its tautomers, salts or solvates thereof.

The use of protecting groups of a certain size, or that are linked simultaneously to two different positions can therefore control the stereochemistry of the product.

Thus the different intermediate compounds of the invention and the starting materials can be prepared using a basic set of simple reactions that allow the protection, functionalization of the different positions in a very stereospecific manner. These procedures will be explained below. Throughout the description and claims the configurations given are only relative configurations.

Enantiomeric compounds of those described in the present application may be prepared using starting materials or reagents (for example, Sharpless dihydroxilation or epoxidation of a double bond) which have the opposite optical rotation. Additionally, conventional techniques for the separation of diastereoisomers and enantiomers are common general knowledge to the skilled person.

As mentioned above, the starting materials of the present invention may be obtained by processes described in PCT/EP2005/005149, PCT/EP2006/068236 and/or PCT/EP2005/005146. Key intermediates described in PCT/EP2005/005146 are benzodienones with the following formula 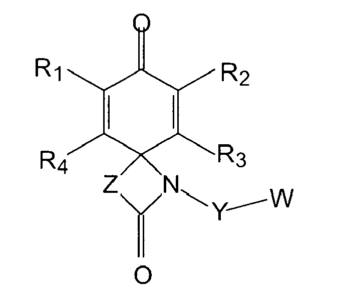 wherein the substitution on position Z may create a stereogenic center. In PCT/EP2005/005146 a preferred embodiment is one wherein Z is -CRaRb-, wherein Ra and Rb are different thus creating a chiral center. Thus, according to a preferred embodiment Ra and Rb in the compounds of formula (I) of the present invention are different, thus creating a chiral center.

Also, PCT/EP2005/005146 discloses as a preferred embodiment compounds wherein the substituent W of the above benzodienones is an aralkyl group, more preferably wherein W is -CRaRb-Q, wherein Ra and Rb in the linker have essentially the same meaning as in the present application and substituent Q has π (pi) interactions with the benzodienone moiety. Thus, another source of chirality arises when Ra and Rb in said linker are different, preferably wherein W is -CHRa-Q (e.g. (-)-(S)-1-(1-Phenylethoxy)-1-azaspiro[3.5]nona-5,8-diene-2,7-dione, which is described as compound 5d in PCT/EP2005/005146). As disclosed in PCT/EP2005/005146 such configuration provides a stereogenic center which allows selectivity or specificity of any further reaction, distinguishing the two double bonds of the of the above mentioned benzodienones, which are intermediates in the synthesis of the compounds of the invention. This will advantageously open the way to diastero- and/or enantioselective synthesis. Therefore, according to a preferred embodiment, W in the compounds of formula (I) of the present invention is -CRcRd-aryl, more preferably Rc and Rd are different, and more preferrably W is -CHRc-aryl.

The above mentioned compound may be a prochiral molecule, for example, if R₁, R₂, R₃ and R₄ are hydrogen. Thus, treatment with chiral reagents will provide the enantiomerically pure intermediate which may drive an enantioselective and diastereoselective synthesis. For example, a Sharpless dihydroxilation or epoxidation will provide the corresponding enantiomerically pure dihydroxyl or epoxide.

PCT/EP2005/005149 also describes the possibility of obtaining chiral compounds following analogous processes.

Therefore, using the chiral materials described in PCT/EP2005/005149, PCT/EP2006/068236 and/or PCT/EP2005/005146, it is possible to obtain the specific stereoisomers and enantiomers of the compounds described in the present invention.

### Definitions

In the present document the following terms have the meaning indicated:
"Alkyl" refers to a straight or branched hydrocarbon chain radical consisting of carbon and hydrogen atoms, containing no saturation, having 1-12, preferably one to eight, more preferably, one to six carbon atoms, and which is attached to the rest of the molecule by a single bond, e. g., methyl, ethyl, n-propyl, i-propyl, n-butyl, t-butyl, n-pentyl, etc.
"Alkenyl" refers to a straight or branched hydrocarbon chain radical consisting of carbon and hydrogen atoms, containing at least one unsaturation, having 2-12, preferably two to eight, preferably two to six carbon atoms, and which is attached to the rest of the molecule by a single bond.
"Alkynyl" refers to a straight or branched hydrocarbon chain radical consisting of carbon and hydrogen atoms, containing at least one carbon-carbon triple bond, conjugated of not, having two to twelve, preferably two to eight six, preferably two to six carbon atoms, and which is attached to the rest of the molecule by a single bond, such as -CCH, -CH₂CCH, -CCCH₃, -CH₂CCCH₃, and which is attached to the rest of the moiecuie by a single bond.
"Aryl" or "Ar" refers to an aromatic hydrocarbon radical such as phenyl, naphthyl or anthracyl.
"Aralkyl" refers to an aryl group linked to an alkyl group such as benzyl and phenethyl.
''Cycloalkyl" refers to a saturated carbocyclic ring having from 3 to 8 carbon atoms.
"Alkoxy" refers to a radical of the formula -Oalkyl, e. g., methoxy, ethoxy, propoxy, etc. "Aryloxy'' refers to a radical of formula -Oaryl.
"Heterocyclyl" refers to a stable 3- to 15- membered ring which consists of carbon atoms and from one to five heteroatoms selected from the group consisting of nitrogen, oxygen, and sulphur, preferably a 4-to 8-membered ring with one or more heteroatoms, more preferably a 5-or 6-membered ring with one or more heteroatoms. For the purposes of this invention, the heterocycle may be a monocyclic, bicyclic or tricyclic ring system, which may include fused ring systems; and the nitrogen, carbon or sulfur atoms in the heterocyclyl radical may be optionally oxidised; the nitrogen atom may be optionally quatemized; and the heterocyclyl radical may be partially or fully saturated or aromatic. Examples of such heterocycles include, but are not limited to, azepines, benzimidazole, benzothiazole, furan, isothiazole, imidazole, indole, piperidine, piperazine, purine, quinoline, thiadiazole, tetrahydrofuran.
"Amino" refers to a radical of the formula-NH₂, -NHR", -NR"R"', wherein R" adn R'" independently represent a group selected from the group consisting of substituted of unsubstituted alkyl, substituted of unsubstituted alkenyl, substituted of unsubstituted alkynyl, substituted of unsubstituted aryl and substituted of unsubstituted aralkyl.
"Hydroxyl protecting group" refers to a group that blocks the OH function for further reactions and can be removed under controlled conditions. The hydroxyl protecting groups are well known in the art, representative protecting groups are:
   - silyl ethers of formula -Si(R')₃, such as trimethylsilyl ether, triethylsilyl ether, tert-butyldimethylsilyl ether, tert-butyldiphenylsilyl ether, tri-isopropylsilyl ether, diethylisopropylsilyl ether, thexyldimethylsilyl ether, triphenylsilyl ether, di-tert-butylmethylsilyl ether;
   - alkyl and arylalkyl ethers such as methyl ether, tert-butyl ether, benzyl ether, p-methoxybenzyl ether , 3,4-dimethoxybenzyl ether, trityl ether; allyl ether;
   - alkoxymethyl and aryloxy ethers of formula -CH₂-O-R', such as methoxymethyl ether, 2-methoxyethoxymethyl ether, benzyloxymethyl ether, p-methoxybenzyloxymethyl ether, 2-(trimethylsilyl)ethoxymethyl ether; tetrahydropyranyl and related ethers; methylthiomethyl ether;
   - Esters of formula -C(=O)R', such as acetate ester, benzoate ester; pivalate ester; methoxyacetate ester; chloroacetate ester; levulinate ester;
   - Carbonates of formula -C(=O)-O-R', such as benzyl carbonate, p-nitrobenzyl carbonate, tert-butyl carbonate, 2,2,2-trichloroethyl carbonate, 2-(trimethylsilyl)ethyl carbonate, allyl carbonate; and
   - sulphates such as SO₃.py.

In all the above formula R' represents a group selected from the group consisting of substituted of unsubstituted alkyl, substituted of unsubstituted alkenyl, substituted of unsubstituted alkynyl, substituted of unsubstituted aryl and substituted of unsubstituted aralkyl. Additional examples of hydroxyl protecting groups can be found in reference books such as Greene and Wuts "Protective Groups in Organic Synthesis'', John Wiley & Sons, Inc., New York, 1999.

"Amino protecting group" refers to a group that blocks the NH₂ function for further reactions and can be removed under controlled conditions. The amino protecting groups are well known in the art, representative protecting groups are carbamates, e.g. carbamates of formula -C(=O)OR'; amides, e.g. amides of formula -C(=O)R', such as substituted or unsubstituted or substituted acetates; or silyl moieties of formula -Si(R')₃; wherein R' represents a group selected from the group consisting of substituted of unsubstituted alkyl, substituted of unsubstituted alkenyl, substituted of unsubstituted alkynyl, substituted of unsubstituted aryl and substituted of unsubstituted aralkyl.. Also different alkyl moeties may serve as amino protecting groups. Said alkyl groups may optionally be substituted by one or more substituents such as halo, hydroxy, alkoxy, alkyloxymethyl ethers, carboxy, cyano, carbonyl, acyl, alkoxycarbonyl, amino, nitro, mercapto and alkylthio. Additional examples of amino protecting groups can be found in reference books such as Greene and Wuts "Protective Groups in Organic Synthesis'', John Wiley & Sons, Inc., New York, 1999.

References herein to substituted groups in the compounds of the present invention refer to the specified moiety that may be substituted at one or more available positions by one or more suitable groups, e. g., halogen such as fluoro, chloro, bromo and iodo ; cyano; hydroxyl ; nitro ; azido ; alkanoyl such as a C₁₋₆ alkanoyl group such as acyl and the like ; carboxamido ; alkyl groups including those groups having 1 to about 12 carbon atoms or from 1 to about 6 carbon atoms and more preferably 1-3 carbon atoms ; alkenyl and alkynyl groups including groups having one or more unsaturated linkages and from 2 to about 12 carbon or from 2 to about 6 carbon atoms ; alkoxy groups having one or more oxygen linkages and from 1 to about 12 carbon atoms or 1 to about 6 carbon atoms ; aryloxy such as phenoxy ; alkylthio groups including those moieties having one or more thioether linkages and from 1 to about 12 carbon atoms or from 1 to about 6 carbon atoms ; alkylsulfinyl groups including those moieties having one or more sulfinyl linkages and from 1 to about 12 carbon atoms or from 1 to about 6 carbon atoms ; alkylsulfonyl groups including those moieties having one or more sulfonyl linkages and from 1 to about 12 carbon atoms or from 1 to about 6 carbon atoms ; aminoalkyl groups such as groups having one or more N atoms and from 1 to about 12 carbon atoms or from 1 to about 6 carbon atoms ; carbocylic aryl having 6 or more carbons, particularly phenyl or naphthyl and aralkyl such as benzyl. Unless otherwise indicated, an optionally substituted group may have a substituent at each substitutable position of the group, and each substitution is independent of the other.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or ¹⁵N-enriched nitrogen are within the scope of this invention.

The invention will be further illustrated by means of examples, which should not be interpreted as limiting the scope of the claims.

### EXAMPLES

### General Methods and Materials

All reactions described below were carried out under argon atmosphere unless otherwise noted. The solvents used were distilled and dried under argon atmosphere before use. All starting materials were purchased commercially (Aldrich, Fluka and Merck) and used without further purification. Flash Chromatography was executed on columns loaded with 230-400 mesh silica gel Merck. TLC was carried out on silica gel Merck (Kieselgel 60F-254).

¹H and ¹³C NMR spectra were obtain on Varian Inova-300 and Varian Inova-400 spectrometers using CDCl₃ as solvent and (CH₃)₄Si as an internal reference unless otherwise noted. ¹H and ¹³C NMR spectral data are reported in parts per million (δ) relative to residual signal of the solvent (CHCl₃, 7.26 ppm and 77.0 ppm for ¹H and ¹³C NMR, respectively). ¹H and ¹³C NMR designations are: s (singlete); br. s (broad singlete); d (doublete); br. d (broad doublete); t (triplete); q (quartete); m (multiplete). Infrared (IR) spectra were record on a Perkin-Elmer FT-IR spectrometer. Low-resolution mass (LRMS) spectra were obtained: (1) on a Hewlett Packard 5973 MSD spectrometer with a direct inlet system (EI) at 70 eV, (2) on a Hewlett Packard LCMS 1100 MSD spectrometer with a chemistry technical by electrospray (API-ES) positive or negative at 4000 V at 330°C and using a mixture [1:1] H₂O/MeOH with 1% AcOH as movil phase.

### Example 1:

### Preparation of rac-(4S,6S,7R,8S,9S)-1-Bencyloxy-7-benzoyloxymethyl-6-tert-butyldimethylsyliloxy-7,8,9-trihydroxy-8,9-O-(1,1,3,3-tetraisopropyldisyloxane-1,3-diyl)-1-azaspiro[3.5]nonane-2,5-dione (1)

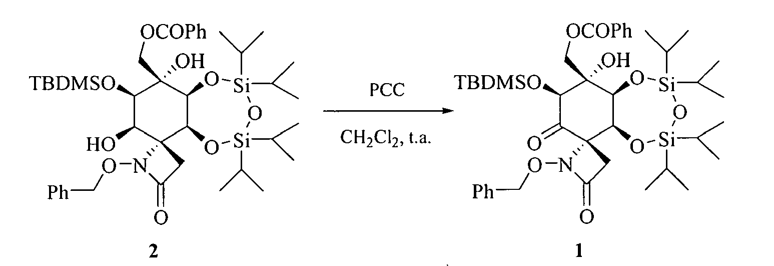

PCC (15 mg, 0,072 mmol, 1.5 eq.) was added to a solution of *rac-(4R,5S,6S,7S,8R,9R)-*1-benzyloxy-7-benzoyloxymethyl-8-tert-butyldimethylsilyloxy-5,6,7,9-tetrahydroxy-5,6-*O*-(1,1,3,3-tetraisopropyldisiloxane-1,3-diyl)-1-azaspiro[3.5]nonan-2-one (2) (39 mg, 0.048 mmol, 1.0 eq.) in CH₂Cl₂ (0,6 ml) and was stirred for 20 hours at room temperature. After that time, a little amount of Celite was added and the solvent was eliminated under reduced pressure. The product was purified by column chromatography (hexane/AcOEt, 10:1) and *rac*-(4*S*,6*S*,7*R*,8*S*,9*S*)-1-benzyloxy-7-benzoyloxymethyl-6-*tert*-butyldimethylsilyloxy-7,8,9-trihydroxy-8,9-*O*-(1,1,3,3-tetraisopropyldisiloxane-1,3-diyl)-1-azaspiro[3.5]nonane-2,5-dione (1) (36 mg. 92% yield) was obtained as a white solid. Also, the starting product 2 (2 mg, 5%) and an isomer of 70 (1mg, 3% yield) was recovered as a white solid.
**P.f.:** 163-164 °C.
***R_{f}*** = 0.53 (TLC, hexano/AcOEt, 3:1).
**¹H-NMR** (200MHz, CDCl₃): δ 8.03 (2H, dm, *J* = 6.9 Hz, Bz), 7.64 (1H, tm, *J* = 7.3 Hz, Bz), 7.49 (2H, ddm, J = 7.3, 6.9 Hz, Bz), 7.44-7.27 (5H, m, Ph), 5.17 (2H, s, OCH₂Ph), 4.85 (1H, d, *J* = 4.2 Hz, H-9), 4.79 (1 H, *part A syst. AB, J* = 11.7 Hz, H-10), 4,57 (1H, dd, *J* = 4.2, 1.3 Hz, H-8), 4.56 (1H, *part B syst. AB, J =* 11.7 Hz, H-10'), 4.12 (1H, d, *J* = 1.3 Hz, H-6), 3.47 (1H, *part A syst. AB, J =* 13.5 Hz, H-3), 2.95 (1 H, s, OH), 2.69 (1H, *part B syst. AB, J* = 13.5 Hz, H-3'), 1.18-0.94 (28H, m, TIPDS), 0.88 (9H, s, C(CH₃)₃), 0.08 (3H, s, SiCH₃), 0.04 (3H, s, SiCH₃).
**¹³C-NMR** (75MHz, CDCl₃): δ 203.1 (C-5), 167.3, 164.7, 135.6, 133.8, 129.7, 128.9, 128.6, 128.2, 78.6, 77.6 (C-6), 75.0 (C-7), 74.6 (C-8), 72.5 (C-4), 68.5 (C-9), 66.6, 39.9 (C-3), 25.6, 18.0, 17.7, 17.4, 17.35, 17.3, 17.2, 17.1, 17.0, 16.9, 13.9, 13.15, 13.1, 13.05, -4.7, -5.6.
**IR** (film): ν 3398, 2947, 2889, 2862, 1763, 1725, 1602, 1588, 1461, 1411, 1385, 1322, 1266, 1150, 1119, 1067, 998, 950, 889, 872, 840, 778 cm⁻¹.
***LRMS** (API-ES⁺):* m/z 1650 (2M+Na)⁺, 836 (M+Na)⁺, 814 (M+H)⁺.
**E.A.** (C₄₁H₆₃NO₁₀Si₃): Found: C, 60.59; H, 7.86; N, 1.95. Calculated: C, 60.48; H, 7.80; N, 1.72.

### Example 2:

### Preparation of rac-(4R, 5S, 6S, 7S, 8S, 9S)-1-Benzyloxi-7-benzoyloxymethyl-8,9-epoxi-5,6,7-trihydroxy-5,6-O-(1,1,3,3-tetraisopropyldisiloxane-1,3-diyl)-1-azaspiro[3.5]nonan-2-one (3)

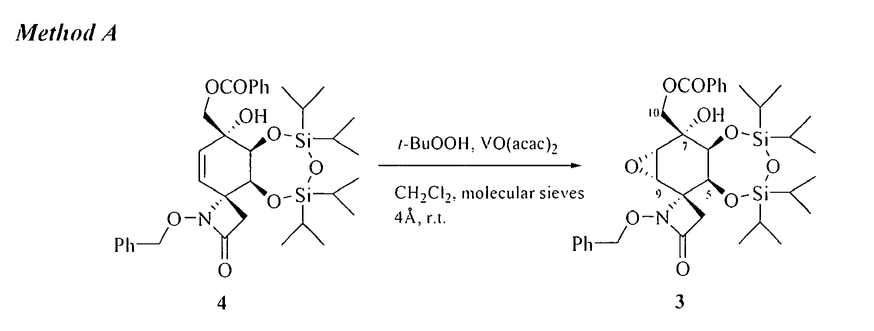

*tert*-butyl hydroperoxide (86 µl, 80% weight solution in (*t*-BuO)₂, 0.689 mmol, 10.0 eq.) and molecular sieves 4Å were added at room temperature to a solution of *rac-*(4*R,* 5*S, 6S,* 7*R*)-1-benzyloxi-7-benzoyloxymethyl-5,6,7-trihydroxy-5,6-*O-*(1,1,3,3,tetraisopropyldisiloxane-1,3-diyl)-1-azaspiro[3.5]non-8-en-2 one (**4**) (46 mg., 0.069 mmol, 1.0 eq.) in CH₂Cl₂ (0.6 ml). After 10 minutes, VO(acac)₂ (5mg, 0.021 mmol, 0,3 eq.) was added. The resultant mixture was stirred at room temperature over 4 days. After that time, de mixture was diluted with CH₂Cl₂ (3 ml) and was filtered under reduced pressure through Celite to eliminate the sieves. The solution was washed with an aqueous solution of NaHCO₃ 10% (2 ml), dried with anhydrous Na₂SO₄, filtered and the solvent was removed under reduced pressure. The product was purified by column chromatography (hexane/AcOEt, 4:1) and *rac-*(4*R,* 5*S*, 6*S,* 7S, 8S, 9*S*)-1-Benzyloxi-7-benzoyloxymethyl-8,9-epoxi-5,6,7-trihydroxy-5,6-*O*-(1,1,3,3-tetraisopropyldisiloxane-1,3-diyl)-1-azaspiro[3.5]nonan-2-one (3) (41 mg, 87% yield) was obtained as a white solid.

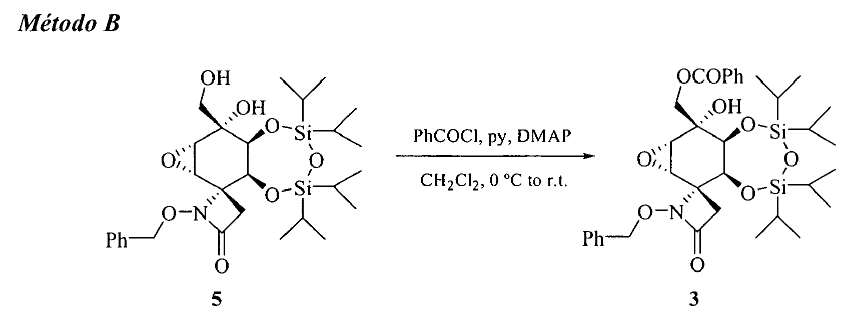

Pyridine (2 µl, 0.024 mmol, 2.0 eq.), a catalytic amount of 4-dimethylaminopyridine (0.01 eq.) and benzoyl chloride (2 µl, 0.014 mmol, 1.4 eq.) were sequentially added at 0°C, to a solution of *rac-(*4*R,* 5*S,* 6*S,* 7*S,* 8*S*, 9*S*)-1-benzyloxi-8,9-epoxi-5,6,7-trihydroxy-7-hydroxymethyl-5,6-*O*-(1,1,3,3-tetraisopropyldisiloxane-1,3-diyl)-1-azaspiro[3.5]nonan-2-one **(5)** (87 mg, 0.012 mmol, 1.0 eq.) in CH₂Cl₂ (0.15 ml). After 18 hours at room temperature, more pyridine (2.0 eq.), DMAP (0.01 eq.) and benzoyl chloride (1.4 eq,) at 0°C were added, and the resulting mixture was stirred at room temperature over 18 hours. After that time, the medium was neutralized by addition of saturated NaHCO₃ and the mixture was extracted with Et₂O (3 x 2ml). The organic phase was washed with HCl 10% (2 ml) and NaCl saturated (2 ml), dried with anhydrous MgSO₄, filtered and the solvent eliminated under reduced pressure. The product was purified by column chromatography (hexane/ Ac=Et, 6:1) obtaining *rac-*(4*R*, 5*S,* 6*S,* 7*S,* 8*S*, 9*S*)-1-Benzyloxi-7-benzoyloxymethyl-8,9-epoxi-5,6,7-trihydroxy-5,6-*O*-(1,1,3,3-tetralsopropyldisiloxane-1,3-diyl)-1-azaspiro[3.5]nonan-2-one **(3)** 85 mg, 62% yield) as a white solid.
***R_{f}**=* 0.36 (TLC, hexane/AcOEt, 3:1); 0.51 (TLC, hexane/AcOEt, 2: 1).
**¹H-NMR** (200MHz, CDCl₃): δ 8.07 (2H, dm, *J=* 7.0 Hz, Bz), 7.60 (1 H, tm, *J* = 7.3 Hz, Bz), 7.53-7.41 (4H, m, Bz y Ph), 7.41-7.31 (3H, m, Ph), 5.12 (1H, *part A syst. AB, J =* 11.0 Hz, OCH₂Ph), 5.03 (1 H, *part B syst. AB, J* = 11.0 Hz, OCH₂Ph), 4.58 (1H, *part A syst. AB, J* = 12.0 Hz, H-10), 4.53 (1H, s broad, H-5), 4.52 (1H, *part B syst. AB, J =* 12.0 Hz, H-10'), 4.24 (1H, s broad, H-6), 3.52 (2H, s broad, H-8 y H-9), 3.16 (1H, *part A syst. AB, J =* 13.9 Hz, H-3), 2.81 (1H, s, OH), 2.48 (1H, *part B syst. AB, J =* 13.9 Hz, H-3'), 1.13-0.98 (28H, m, TIPDS).
**¹H-NMR** (300MHz, C₆D₆): δ 8.16 (2H, dm, *J* = 8.3 Hz, Bz), 7.50 (1H, d, *J* = 7.6 Hz, Bz), 7.20-7.01 (7H, m, Bz y Ph), 5.10 (1H, *part A syst. AB, J=* 11.5 Hz, OCH₂Ph), 5.06 (1H, *part B syst. AB, J =* 11.5 Hz, OCH₂Ph), 4.76 (1 H, t, *J =* 1.9 Hz, H-5 o H-6), 4.52 (1H, *part A syst. AB, J =* 11.7 Hz, H-10), 4.45 (1H, *part B syst. AB, J =* 11.7 Hz, H-10'), 4.28 (1H, m, H-6 o H-5), 3.17 (1H, d, *J*= 13.7 Hz, H-3), 3.02 (1H, m, H-8 o H-9), 2.96 (1H, dd, *J* = 3.7, 1.9 Hz, H-9 o H-8), 2.73 (1H, m, OH), 2.18 (1 H, dd, *J =* 13.7, 1.9 Hz, H-3'), 1.35-0.80 (28H, m, TIPDS).
**¹H-RMN** (500MHz, CO(CD₃)₂): δ 8.12 (2H, m, Bz), 7.66 (1H, m, Bz), 7.57-7.46 (4H, m, Bz y Ph), 7.42-7.32 (3H, m, Ph), 5.12 (1H, *part A syst. AB, J =* 10.9 Hz, OCH₂Ph), 5.06 (1H, *part B syst. AB, J =* 10.9 Hz, OCH₂Ph), 4.98 (1H, s, OH), 4.88 (1H, d, *J =* 2.4 Hz, H-5), 4.59 (1H, *part A syst. AB, J =* 11.5 Hz, H-10), 4.54 (1H, *part B syst. AB, J =* 11.5 Hz, H-10'), 4.37 (1H, dd, *J*= 2.4, 1.3 Hz, H-6), 3.71 (1H, d, *J* = 3.7 Hz, H-9), 3.64 (1H, dd, *J* = 3.7, 1.3 Hz, H-8), 3.08 (1H, *part A syst. A B, J =* 13.5 Hz, H-3), 2.68 (1 H. *part B syst. AB, J=* 13.5 Hz, H-3'), 1.23-0.92 (28H, m, TIPDS).
**¹³C-RMN** (75MHz, CDCl₃): δ 166.4, 163.9, 135.2, 133.4, 129.6, 129.5, 128.9, 128.7. 128.5, 128.4, 78.8, 76.6 (C-6), 71.4 (C-7), 66.8 (C-10), 66.2 (C-5), 65.5 (C-4), 60.3 (C-9), 55.0 (C-8), 38.3 (C-3), 17.6, 17.5, 17.2, 17.1, 17.0, 16.9, 14.3, 14.2, 13.5, 13.2.
**¹³C-RMN** (125MHz, CO(CD₃)₂): δ 166.9, 164.4, 136.9, 134.2, 131.2, 130.5, 129.8, 129.5, 129.4, 129.3, 79.2, 78.5 (C-6), 72.3 (C-7), 68.7 (C-10), 67.8 (C-5), 66.6 (C-4), 59.8 (C-9), 56.2 (C-8), 38.8 (C-3), 18.2, 18.1, 17.7, 17.6, 17.55, 17.5, 15.4, 15.2, 14.4, 14.0.
**IR** (film): ν 3430, 2947, 2900, 2868, 1767, 1725, 1599, 1589, 1465, 1451, 1394, 1370, 1273, 1148, 1104, 1075, 1007, 941, 886, 823, 756, 711 cm⁻¹.
***LRMS** (API-ES⁺):* m/z 1389 (2M+Na)⁺, 706 (M+Na)⁺, 684 (M+H)⁺.
A.E. (C₃₅H₄₉NO₉Si₂): Found: C, 61.54; H, 7.37; N, 2.21. Calculated: C, 61.46; H, 7.22; N, 2.05.

### Example 3:

### Preparation of rac-(4S,5R,6R,7R,8S,9S)-1-Bencyloxy-7-benzoyloxymethyl-6-tert-butyldimethylsyliloxy-5-cyano-8,9-dihydroxy-8,9-O-(1,1,3,3-tetraisopropyldisiloxane-1,3-diil)-5,7-bis(methoxycarbonoyloxy)-1-azaspiro[3.5]nonan-2-one (6)

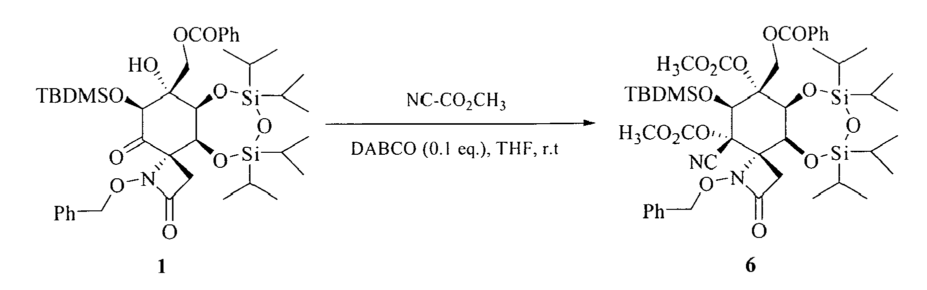

To a solution of *rac*-(4*S*,6*S*,7*R*,8*S*,9*S*)-1-bencyloxy-7-benzoyloxymethyl-6-*tert*-butyldimethylsyliloxy-7,8,9-trihydroxy-8,9-*O*-(1,1,3,3-tetraisopropyldisyloxane-1,3-diil)-1-azaspiro[3.5]nonane-2,5-dione **(1)** (85 mg, 0,104 mmol, 1.0 eq.) and methyl cyanoformiate (100 µl, 1.248 mmol, 12.0 eq.) in THF (1 ml) DABCO was added at room temperature (3 mg, 0.026 mmol, 0.25 eq.). The resulting mixture was stirred at room temperature for 15 hours, after which solvent was removed under reduced pressure. The residue was grinded with AcOEt, filtered through Celite and the solvent removed under reduced pressure. The product was purified by silica gel column chromatography (hexane/AcOEt, 8:1) and *rac-*(*4S,5R,6R,7R,8S,9S)*-1-Bencyloxy-7-benzoyloxymethyl-6-*tert*-butyldimethylsyliloxy-5-cyano-8,9-dihydroxy-8,9-*O*-(1,1,3,3-tetraisopropyldisiloxane-1,3-diil)-5,7-bis(methoxycarbonoyloxy)-1-azaspiro[3.5]nonan-2-one **(6)** (29 mg, yield. 29%) was obtained as a white solid. An isomer was also isolated (5 mg, rto. 5%, *R_{f}=* 0.49 (TLC, hexane/AcOEt, 3:1)).
***R_{f}** =* 0.41 (TLC, hexane/AcOEt, 3: 1).
**¹H-NMR** (300MHz, CDCl₃): δ 7.98 (2H, , J = 6.9 Hz, Bz), 7.61 (1H,, *J*= 7.4 Hz, Bz), 7.53-7.41 (4H, m, Bz y Ph), 7.41-7.28 (3H, m, Ph), 5.56 (1H, d, *J* = 1.3 Hz, H-6), 5.14 (1H, dd, *J =* 4.0, 1.3 Hz, H-8), 5.08 (1H, *part A syst. AB, J =* 12.0 Hz, H-10), 5.05 (1H, *pare A syst. AB, J* = 10.1 Hz, OCH₂Ph), 5.00 (1H, *part B syst. AB, J* = 10.1 Hz, OCH₂Ph), 4.88 (1H, d, *J* = 4.0 Hz, H-9), 4.83 (1H, *part B syst. AB, J=* 12.0 Hz, H-10'), 3.90 (3H, s, OCH₃), 3.62 (3H, s, OCH₃), 3.45 (1H, *part A syst. AB, J* = 14.8 Hz, H-3), 3.05 (1 H, *part B syst. AB, J* = 14.8 Hz, H-3'), 1.16-0.88 (28H, m, TIPDS), 0.95 (9H, s, C(CH₃)₃), 0.40 (3H, s, SiCH₃), 0.23 (3H, s, SiCH₃).
**¹³C-NMR** (75MHz, CDCl₃): δ 167.0, 165.4, 152.9, 152.7, 135.3, 133.5, 129.4, 129.2, 128.9, 128.6, 128.3, 128.2, 115.1 (CN), 83.6 (C-7), 81.9 (C-5), 78.7, 71.0 (C-8), 68.7 (C-6), 66.5 (C-4), 65.2 (C-9), 61.9 (C-10), 55.8, 55.1, 38.8 (C-3), 26.4, 18.6, 17.6, 17.4, 17.3, 17.1, 17.0, 16.9, 16.8, 16.7, 13.5, 13.3, 13.1, 13.0, -3.3, -4.0.
**IR** (KBr): ν 3436, 2946, 2889, 2862, 2338, 1794, 1776, 1751, 1729, 1633, 1465, 1440, 1394, 1378, 1293, 1263, 1158, 1121, 1089, 1044, 995, 977, 922, 840, 785, 712 cm⁻¹. ***LRMS** (API-ES⁺):* m/z 1936 (2M+Na)⁺, 1915 (2M+H)⁺, 1029 (M+73)⁺, 957 (M+H)⁺.

### Examples for General Formula II and further epimerization (Compounds 7, 8, 11 and 14)

### Example 4

### Preparation of rac-(4R,5R,6R,7S,8S,9S)-1-benzyloxy-7-(benzoyloxymethyl)-6-(tert-butyldimethylsilyloxy)-5-cyano-5,7,8,9-tetrahydroxy -8,9-O-(1,1,3,3-tetraisopropyldisiloxane-1,3-diyl)-1-azaspiro[3.5]nonan-2-one (7) and rac-(4R,5R,6R,7S,8S,9S)-1-benzyloxy-6-(tert-butyldimethylsilyloxy)-5-cyano-5,7,8,9-tetrahydroxy-8,9-O-(1,1,3,3-tetraisopropyldisiloxane-1,3-diyl)-7-methylhydroxy-1-azaspiro(3.5)nonan-2-one (8)

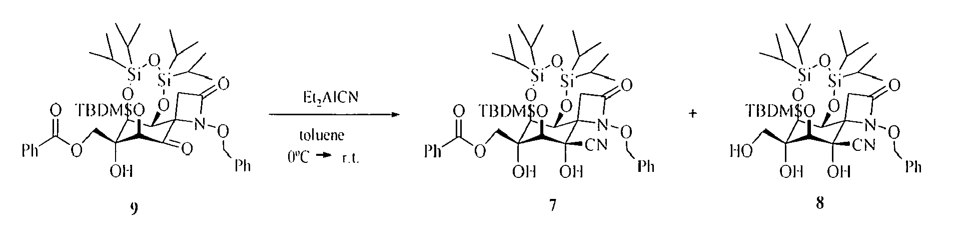

To a stirred solution of *rac*-(4*S*,6*S*,7*R*,8*S*,9*S*)-1-bencyloxy-7-benzoyloxymethyl-6-*tert-*butyldimethylsilyloxy-7,8,9-trihydroxy-8,9-*O*-(1,1,3,3-tetraisopropyldisiloxane-1,3-diyl)-1-azaespiro[3.5]nonan-2,5-dione **(9)** (92 mg, 0.113 mmol) in toluene (1 ml) at 0° C was added a solution of diethylaluminiumcyanide in toluene (1.0 M, 0.23 ml, 0.227 mmol). The resulting mixture was stirred at room temperature until the reaction was complete (24 h, TLC monitoring, hexane/AcOEt, 3:1). The reaction was quenched at room temperature with a mixture 10% aqueous potassium-sodium tartrate solution-AcOEt (1:1, 4ml). The layers were separated and aqueous phase was extracted with AcOEt (3 x 4 ml). The combined organic extracts were dried over Na₂SO₄, filtered and concentrated under reduced pressure to give *rac-*(4*R*, *5R*,6*R*,7*S*,8*S*,9*S*)-1-benzyloxy-7-(benzoyloxymethyl)-6-(tert-butyldimethylsilyloxy)-5-cyano-5,7,8,9-tetrahydroxy-8,9-*O*-(1,1,3,3-tetraisopropyldisiloxane-1,3-diyl)-1-azaspiro[3.5]nonan-2-one (**7**) (79 mg, 83%) as a white solid and *rac-*(4*R,*5*R,*6*R,*7*S,*8*S,*9*S)-*1-benzyloxy-6-(tert-butyldimethylsilyloxy)-5-cyano-5,7,8,9-tetrahydroxy-8,9-*O*-(1,1,3,3-tetraisopropyldisiloxane-1,3-diyl)-7-methylhydroxy-1-azaspiro[3.5]nonan-2-one (**8**) (10 mg, 10%) as a white solid. 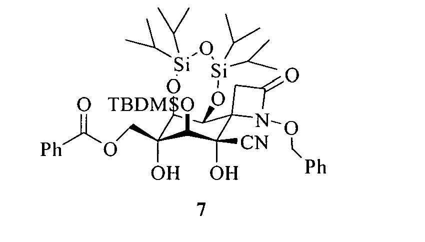 *R_{f}* = 0.38 (TLC, hexane/AcOEt 3:1) ; **yield,** 83% ; white solid ; **m.p**. :160-162° C; **¹H-NMR** (300 MHz CDCl₃): δ 8.05 (2H, d, J = 8.3Hz, Bz), 7.66 (1H, m, Bz), 7.51 (2H, m, Bz), 7.44-7.26 (5H, m, Ph), 5.33 (1H, br. s, OH), 5.16 (1H, *part A* syst. *AB,* J = 10.8 Hz, OCH₂Ph), 5.11 (1H, *part B syst. AB,* J = 10.8 Hz, OCH₂Ph), 4.91 (1H, d, J = 4.1 Hz, H-9), 4.75 (1H, br. s, OH), 4.71 (1H; *part A syst. AB,* J = 12.2Hz, H-10), 4.67 (1H, *part B syst. AB,* J = 12.2 Hz, H- 10'), 4.52 (1H, dd, J = 4.0, 1.5 Hz, H-8), 4.27 (1H, d , J = 1.5 Hz, H-6), 3.31 (1H, *part A syst. AB,* J = 14.7 Hz, H-3), 2.87 (1H, *part B syst. AB,* J = 14.7 Hz, H-3'), 1.08-1.01, (28H, m, TIPDS), 0.96 (9H, s, C(CH₃)₃), 0.34 (3H, s, SiCH₃), 0.28 (3H, s, SiCH₃); **¹³C-NMR** (75 MHz, CDCl₃):168.7, 167.2, 135.7, 134.3, 129.9, 128.82, 128.80, 128.4, 128.3, 118.1, 79.3, 78.5, 77.3, 75.4, 74.0, 67.9, 67.8, 64.9, 38.6, 26.5, 18.8, 17.8, 17.4, 17.31, 17.26, 17.15, 17.05, 16.8, 13.8, 13.07, 13.05, 13.04, 1.0, - 3.8, -4.2; **LMRS(API-ES⁺):** m/z 841 (M+H)⁺, 842 (M+2H)⁺, 843 (M+3H)⁺, 863(M+Na)⁺, 1705 (2M+Na+H)⁺; **IR** (film): ν 3333.3, 2947.0, 2866.0, 2348.9, 1759.3, 1726.6, 1462.5, 1453.2, 1263.6, 1154.9, 1113.0, 1067.9, 999.6, 838.0, 779.0 cm⁻¹.

### X-ray Diffaction of rac-(4R,5R,6R,7S,8S,9S)-1-benzyloxy-7-(benzoyloxymethyl)-6-(tert-butyldimethylsilyloxy)-5-cyano-5,7,8,9-tetrahydroxy-8,9-O-(1,1,3,3-tetraisopropyldisiloxane-1,3-diyl)-1-azaspiro[3.5]nonan-2-one (7)

A solution of *rac*-(4*R*,5*R*,6*R*,7*S*,8*S*,9*S*)-1-benzyloxy-7-(benzoyloxymethyl)-6-(tert-butyldimethylsilyloxy)-5-cyano-5,7,8,9-tetrahydroxy-8,9-*O*-(1,1,3,3-tetraisopropyldisiloxane-1,3-diyl)-1-azaspiro[3.5]nonan-2-one (7) in AcOEt (24 mM, 0.5 ml) was crystallize by hexane diffusion.

Crystal structure determination was carried out using a Bruker-Nonius diffractometer equipped with a APPEX 2 4K CCD area detector, a FR591 rotating anode with Mo_{Kα} radiation, Montel mirrors as monochromator and a Kryoflex low temperature device (T = 100 K). Fullsphere data collection omega and phi scans. Programs used: Data collection Apex2 V. 1.0-22 (Bruker-Nonius 2004), data reduction Saint + Version 6.22 (Bruker-Nonius 2001) and absorption correction SADABS V. 2.10 (2003). Crystal structure solution was achieved using direct methods as implemented in SHELXTL Version 6.10 (Sheldrick, Universtität Göttingen (Germany), 2000) and visualized using XP program. Missing atoms were subsequently located from difference Fourier synthesis and added to the atom list. Least-squares refinement on F2 using all measured intensities was carried out using the program SHELXTL Version 6.10 (Sheldrick, Universtität Göttingen (Germany), 2000). All non hydrogen atoms were refined including anisotropic displacement parameters. Crystallographic data and experimental details for structural analyses are summarized in Table 1, whereas positional parameters and atomic coordinates are given in Table 2 and Table 3. Figure 1 is a representation of the molecular structure obtained using X-rays diffraction.

**Table 1. Crystal data and structure**

| | |
|---|---|
| Empirical formula | C₄₂H₆₄N₂O₁₀Si₃ |
| Formula weight | 841.22 |
| Temperature | 100(2) K |
| Wavelength (Å) | 0.71073 |
| Crystal system, space group | Monoclinic, P21/n |
| Unit cell dimensions | a = 11.9912 (10) Å a= 90° |
| | b =14.7024 (13) Å b= 94.365(2)° |
| | c = 25.768(2)) Å g = 90° |
| Volume | 4529.7(7) Å³ |
| Z | 4 |
| Calculated density | 1.142 Mg/m³ |
| µ (mm⁻¹) Absortion coefficient | 0.149 mm⁻¹ |
| F(000) | 1808 |
| Crystal size | 0.40 x 0.30 x 0.20 mm³ |
| θ range for data collection | 2.75 to 39.60° |
| Index ranges | -21≤h≤-12≤k≤26,46≤I≤46 |
| Reflections collected | 90625 |
| Independent reflections | 26303 [R(int) = 0.0463] |
| Completeness to theta = 39.60° | 96.1% |
| Absorption correction | SADABS (Bruker-Nonius) |
| Max. and min. transmission | 0.9686 and 0.9386 |
| Refinement method | Full-matrix least-squares o n F² |
| Data / restraints / parameters | 26303 / 0 / 598 |
| Goodness-of-fit on F² | 1.034 |
| Final R indices [1>2sigma(I)] | R1 = 0.0514, wR2 = 0.1380 |
| R indices (all data) | R1 = 0.0677, wR2 = 0.1533 |
| Largest diff. peak and hole | 0.943 and -0.742 e. Å⁻³ |

**Table 2. Bond lengths [Å] and angles [°]**

| | | | | | |
|---|---|---|---|---|---|
| Si(1)- | 1.6358(6) | C(1)-O(2) | 1.4195(8) | C(13) | |
| O(1) | | C(1)-C(2) | 1.5378(9) | Si(2)- | 1.8721(10) |
| Si(1)-O(2) | 1.6712(6) | C(1)-C(3) | 1.5447(10) | C(17) | |
| Si(1)-C(7) | 1.8662(9) | N(1)- | 1.1492(13) | C(2)-O(3) | 1.4142(8) |
| Si(1)- | 1.8798(8) | C(27) | | C(2)-C(6) | 1.5305(10) |
| C(10) | | Si(2)-O(3) | 1.6557(6) | N(2)- | 1.3702(11) |
| O(1)-Si(2) | 1.6420(6) | Si(2)- | 1.8659(9) | C(29) | |
| N(2)-O(9) | 1.3842(9) | C(7)-C(8) | 1.5387(13) | C(31) | |
| N(2)-C(6) | 1.4861(9) | O(7)- | 1.2120(11) | C(31)- | 1.3930(12) |
| C(3)-O(4) | 1.4328(9) | C(20) | | C(36) | |
| C(3)- | 1.5341(11) | O(8)- | 1.2172(10) | C(31)- | 1.3940(14) |
| C(19) | | C(29) | | C(32) | |
| C(3)-C(4) | 1.5564(11) | O(9)- | 1.4535(10) | C(32)- | 1.3974(17) |
| Si(3)- | 1.6854(7) | C(30) | | C(33) | |
| O(10) | | C(10)- | 1.5359(13) | C(33)- | 1.3900(18) |
| Si(3)- | 1.863(2) | C(11) | | C(34) | |
| C(38) | | C(10)- | 1.5396(14) | C(34)- | 1.384(2) |
| Si(3)- | 1.876(2) | C(12) | | C(35) | |
| C(37) | | C(13)- | 1.5313(15) | C(35)- | 1.3942(16) |
| Si(3)- | 1.876(5) | C(15) | | C(36) | |
| C(39) | | C(13)- | 1.5407(17) | C(39)- | 1.534(4) |
| Si(3')- | 1.7189(7) | C(14) | | C(41) | |
| O(10) | | C(16)- | 1.5411(15) | C(39)- | 1.539(5) |
| Si(3')- | 1.858(2) | C(17) | | C(40) | |
| C(38') | | C(17)- | 1.5325(15) | C(39)- | 1.566(10) |
| Si(3')- | 1.876(2) | C(18) | | C(42) | |
| C(37') | | C(20)- | 1.4868(11) | C(39')- | 1.501(10) |
| Si(3')- | 1.878(6) | C(21) | | C(42') | |
| C(39') | | C(21)- | 1.3950(12) | C(39')- | 1.504(7) |
| C(4)- | 1.4098(9) | C(26) | | C(41') | |
| O(10) | | C(21)- | 1.3998(11) | C(39')- | 1.524(7) |
| C(4)-C(5) | 1.5601(11) | C(22) | 1.3946(13) | C(40') | |
| C(5)-O(5) | 1.4219(9) | C(22)- | | | |
| C(5)- | 1.4872(12) | C(23) | | | |
| C(27) | | C(23)- | 1.3915(15) | | |
| C(5)-C(6) | 1.5452(10) | C(24) | | | |
| C(6)- | 1.5575(10) | C(24)- | 1.3896(15) 5 | | |
| C(28) | | C(25) | | | |
| O(6)- | 1.3581(11) | C(25)- | 1.3927(14) | | |
| C(20) | | C(26) | | | |
| O(6)- | 1.4370(10) | C(28)- | 1.5315(11) | | |
| C(19) | | C(29) | | | |
| C(7)-C(9) | 1.5322(15) | C(30)- | 1.5033(13) | | |
| | | | | | |
| O(1)-Si(1)-O(2) | | 107.81(3) | O(1)-Si(1)-C(10) | | 107.58(4) |
| O(1)-Si(1)-C(7) | | 111.13(4) | O(2)-Si(1)-C(10) | | 111.65(3) |
| O(2)-Si(1)-C(7) | | 103.22(3) | C(7)-Si(1)-C(10) | | 115.24(4) |
| Si(1)-O(1)-Si(2) | | 134.92(4) | C(27)-C(5)-C(6) | | 109.18(7) |
| O(2)-C(1)-C(2) | | 111.75(6) | O(5)-C(5)-C(4) | | 111.25(6) |
| O(2)-C(1)-C(3) | | 109.90(5) | C(27)-C(5)-C(4) | | 109.52(7) |
| C(2)-C(1)-C(3) | | 112.44(5) | C(6)-C(5)-C(4) | | 111.21(6) |
| O(1)-Si(2)-O(3) | | 108.19(3) | N(2)-C(6)-C(2) | | 110.94(5) |
| O(1)-Si(2)-C(13) | | 108.05(4) | N(2)-C(6)-C(5) | | 113.04(6) |
| O(3)-Si(2)-C(13) | | 104.61(4) | C(2)-C(6)-C(5) | | 110.36(6) |
| O(1)-Si(2)-C(17) | | 106.28(4) | N(2)-C(6)-C(28) | | 85.95(5) |
| O(3)-Si(2)-C(17) | | 111.90(4) | C(2)-C(6)-C(28) | | 115.65(6) |
| C(13)-Si(2)-C(17) | | 117.49(4) | C(5)-C(6)-C(28) | | 118.65(6) |
| C(1)-O(2)-Si(1) | | 121.94(4) | C(20)-O(6)-C(19) | | 114.78(7) |
| O(3)-C(2)-C(6) | | 107.16(6) | C(9)-C(7)-C(8) | | 110.81 (8) |
| O(3)-C(2)-C(1) | | 112.68(5) | C(9)-C(7)-Si(1) | | 112.65(7) |
| C(6)-C(2)-C(1) | | 112.47(5) | C(8)-C(7)-Si(1) | | 115.42(7) |
| C(29)-N(2)-O(9) | | 125.28(7) | N(2)-O(9)-C(30) | | 110.41(6) |
| C(29)-N(2)-C(6) | | 95.06(6) | C(11)-C(10)-C(12) | | 109.02(8) |
| O(9)-N(2)-C(6) | | 124.86(6) | C(11)-C(10)-Si(1) | | 110.35(7) |
| C(2)-O(3)-Si(2) | | 129.79(5) | C(12)-C(10)-Si(1) | | 114.81(6) |
| O(4)-C(3)-C(19) | | 110.50(6) | C(4)-O(10)-Si(3) | | 129.91(5) |
| O(4)-C(3)-C(1) | | 109.40(6) | C(4)-O(10)-Si(3') | | 122.21(5) |
| C(19)-C(3)-C(1) | | 107.34(6) | Si(3)-O(10)-Si(3') | | 23.60(2) |
| O(4)-C(3)-C(4) | | 103.07(6) | C(15)-C(13)-C(14) | | 109.46(9) |
| C(19)-C(3)-C(4) | | 111.86(7) | C(15)-C(13)-Si(2) | | 113.98(8) |
| C(1)-C(3)-C(4) | | 114.63(6) | C(14)-C(13)-Si(2) | | 109.99(6) |
| O(10)-Si(3)-C(38) | | 106.57(7) | C(18)-C(17)-C(16) | | 111.29(9) |
| O(10)-Si(3)-C(37) | | 115.66(7) | C(18)-C(17)-Si(2) | | 115.44(8) |
| C(38)-Si(3)-C(37) | | 109.41(11) | C(16)-C(17)-Si(2) | | 110.07(8) |
| O(10)-Si(3)-C(39) | | 105.15(14) | O(6)-C(19)-C(3) | | 110.39(7) |
| C(38)-Si(3)-C(39) | | 112.18(14) | O(7)-C(20)-O(6) | | 123.36(8) |
| C(37)-Si(3)-C(39) | | 107.91(14) | O(7)-C(20)-C(21) | | 124.07(8) |
| O(10)-Si(3')-C(38') | | 108.22(7) | O(6)-C(20)-C(21) | | 112.56(7) |
| O(10)-Si(3')-C(37') | | 116.06(8) | C(26)-C(21)-C(22) | | 120.19(8) |
| C(38')-Si(3')-C(37') | | 105.47(15) | C(26)-C(21)-C(20) | | 117.21 (7) |
| O(10)-Si(3')-C(39') | | 104.04(18) | C(22)-C(21)-C(20) | | 122.56(7) |
| C(38')-Si(3')-C(39') | | 112.12(18) | C(23)-C(22)-C(21) | | 119.71(8) |
| C(37')-Si(3')-C(39') | | 111.1(2) | C(24)-C(23)-C(22) | | 119.85(9) |
| O(10)-C(4)-C(3) | | 112.92(7) | C(25)-C(24)-C(23) | | 120.45(9) |
| O(10)-C(4)-C(5) | | 109.20(6) | C(24)-C(25)-C(26) | | 120.05(9) |
| C(3)-C(4)-C(5) | | 109.79(5) | C(25)-C(26)-C(21) | | 119.74(9) |
| O(5)-C(5)-C(27) | | 105.40(6) | N(1)-C(27)-C(5) | | 178.24(13) |
| O(5)-C(5)-C(6) | | 110.11(6) | C(29)-C(28)-C(6) | | 86.08(5) |
| O(8)-C(29)-N(2) | | 132.48(8) | C(41)-C(39)-C(40) | | 108.5(3) |
| O(8)-C(29)-C(28) | | 136.34(8) | C(4 1)-C(39)-C(42) | | 107.5(3) |
| N(2)-C(29)-C(28) | | 91.16(6) | C(40)-C(39)-C(42) | | 109.6(4) |
| O(9)-C(30)-C(31) | | 106.72(7) | C(41)-C(39)-Si(3) | | 112.9(3) |
| C(36)-C(31)-C(32) | | 119.01(9) | C(40)-C(39)-Si(3) | | 110.6(2) |
| C(36)-C(31)-C(30) | | 120.44(9) | C(42)-C(39)-Si(3) | | 107.7(4) |
| C(32)-C(31)-C(30) | | 120.55(8) | C(42')-C(39')-C(41') | | 110.3(5) |
| C(31)-C(32)-C(33) | | 120.52(10) | C(42')-C(39')-C(40') | | 107.0(5) |
| C(34)-C(33)-C(32) | | 119.97(11) | C(41')-C(39')-C(40') | | 108.4(6) |
| C(35)-C(34)-C(33) | | 119.67(10) | C(42')-C(39')-Si(3') | | 110.8(5) |
| C(34)-C(35)-C(36) | | 120.49(10) | C(41')-C(39')-Si(3') | | 109.9(4) |
| C(31)-C(36)-C(35) | | 120.32(10) | C(40')-C(39')-Si(3') | | 110.3(4) |

**Table 3. Torsion angles [°]**

| | | | |
|---|---|---|---|
| O(2)-Si(1)-O(1)-Si(2) | 6.76(7) | O(4)-C(3)-C(4)-O(10) | 169.08(6) |
| C(7)-Si(1)-O(1)-Si(2) | -105.68(6) | C(19)-C(3)-C(4)-O(10) | 50.36(8) |
| C(10)-Si(1)-O(1)-Si(2) | 127.30(6) | C(1)-C(3)-C(4)-O(10) | -72.13(8) |
| Si(1)-O(1)-Si(2)-O(3) | 10.22(7) | O(4)-C(3)-C(4)-C(5) | -68.80(7) |
| Si(1)-O(1)-Si(2)-C(13) | 122.95(6) | C(19)-C(3)-C(4)-C(5) | 172.48(6) |
| Si(1)-O(1)-Si(2)-C(17) | -110.11(6) | C(1)-C(3)-C(4)-C(5) | 49.99(8) |
| C(2)-C(1)-O(2)-Si(1) | -96.90(6) | O(10)-C(4)-C(5)-O(5) | -168.11(6) |
| C(3)-C(1)-O(2)-Si(1) | 137.54(5) | C(3)-C(4)-C(5)-O(5) | 67.59(7) |
| O(1)-Si(1)-O(2)-C(1) | 45.00(6) | O(10)-C(4)-C(5)-C(27) | -52.00(8) |
| C(7)-Si(1)-O(2)-C(1) | 162.67(6) | C(3)-C(4)-C(5)-C(27) | -176.30(6) |
| C(10)-Si(1)-O(2)-C(1) | -72.96(6) | O(10)-C(4)-C(5)-C(6) | 68.76(8) |
| O(2)-C(1)-C(2)-O(3) | 47.74(8) | C(3)-C(4)-C(5)-C(6) | -55.54(8) |
| C(3)-C(1)-C(2)-O(3) | 171.88(6) | C(29)-N(2)-C(6)-C(2) | -105.61(6) |
| O(2)-C(1)-C(2)-C(6) | -73.52(7) | O(9)-N(2)-C(6)-C(2) | 34.60(9) |
| C(3)-C(1)-C(2)-C(6) | 50.62(8) | C(29)-N(2)-C(6)-C(5) | 129.82(6) |
| C(6)-C(2)-O(3)-Si(2) | 167.26(5) | O(9)-N(2)-C(6)-C(5) | -89.97(8) |
| C(1)-C(2)-O(3)-Si(2) | 43.03(8) | C(29)-N(2)-C(6)-C(28) | 10.34(6) |
| O(1)-Si(2)-O(3)-C(2) | -66.37(7) | O(9)-N(2)-C(6)-C(28) | 150.54(7) |
| C(13)-Si(2)-O(3)-C(2) | 178.62(6) | O(3)-C(2)-C(6)-N(2) | 52.72(7) |
| C(17)-Si(2)-O(3)-C(2) | 50.38(7) | C(1)-C(2)-C(6)-N(2) | 177.08(6) |
| O(2)-C(1)-C(3)-O(4) | -167.52(6) | O(3)-C(2)-C(6)-C(5) | 178.80(5) |
| C(2)-C(1)-C(3)-O(4) | 67.32(8) | C(1)-C(2)-C(6)-C(5) | -56.84(7) |
| O(2)-C(1)-C(3)-C(19) | -47.59(8) | O(3)-C(2)-C(6)-C(28) | -43.01(7) |
| C(2)-C(1)-C(3)-C(19) | -172.75(6) | C(1)-C(2)-C(6)-C(28) | 81.35(7) |
| O(2)-C(1)-C(3)-C(4) | 77.32(7) | O(5)-C(5)-C(6)-N(2) | 60.87(8) |
| C(2)-C(1)-C(3)-C(4) | -47.84(8) | C(27)-C(5)-C(6)-N(2) | -54.39(8) |
| C(4)-C(5)-C(6)-N(2) | -175.35(6) | Si(3) | |
| O(5)-C(5)-C(6)-C(2) | -64.02(7) | C(37')-Si(3')-O(10)- | -172.25(17) |
| C(27)-C(5)-C(6)-C(2) | -179.28(6) | Si(3) | |
| C(4)-C(5)-C(6)-C(2) | 59.76(7) | C(39')-Si(3')-O(10)- | 65.41(17) |
| O(5)-C(5)-C(6)-C(28) | 159.20(6) | Si(3) | |
| C(27)-C(5)-C(6)-C(28) | 43.94(9) | O(1)-Si(2)-C(13)-C(15) | 173.08(8) |
| C(4)-C(5)-C(6)-C(28) | -77.02(8) | O(3)-Si(2)-C(13)-C(15) | -71.81(9) |
| O(1)-Si(1)-C(7)-C(9) | -171.19(7) | C(17)-Si(2)-C(13)- | 52.95(9) |
| O(2)-Si(1)-C(7)-C(9) | 73.49(8) | C(15) | |
| C(10)-Si(1)-C(7)-C(9) | -48.50(8) | O(1)-Si(2)-C(13)-C(14) | -63.55(7) |
| O(1)-Si(1)-C(7)-C(8) | -42.53(8) | O(3)-Si(2)-C(13)-C(14) | 51.55(7) |
| O(2)-Si(1)-C(7)-C(8) | -157.85(7) | C(17)-Si(2)-C(13)- | 176.32(7) |
| C(10)-Si(1)-C(7)-C(8) | 80.16(8) | C(14) | |
| C(29)-N(2)-O(9)-C(30) | -119.66(8) | O(1)-Si(2)-C(17)-C(18) | 170.99(7) |
| C(6)-N(2)-O(9)-C(30) | 111.69(8) | O(3)-Si(2)-C(17)-C(18) | 53.09(8) |
| O(1)-Si(1)-C(10)-C(11) | 77.42(8) | C(13)-Si(2)-C(17)- | -67.95(9) |
| O(2)-Si(I)-C(10)-C(11) | -164.49(7) | C(18) | |
| C(7)-Si(1)-C(10)-C(11) | -47.15(8) | O(1)-Si(2)-C(17)-C(16) | -62.00(8) |
| O(i)-Si(1)-C(10)-C(12) | -46.25(7) | O(3)-Si(2)-C(17)-C(16) | -179.90(7) |
| O(2)-Si(1)-C(10)-C(12) | 71.84(7) | C(13)-Si(2)-C(17)- | 59.06(9) |
| C(7)-Si(1)-C(10)-C(12) | -170.82(7) | C(16) | |
| C(3)-C(4)-O(10)-Si(3) | -98.37(8) | C(20)-O(6)-C(19)-C(3) | 97.88(9) |
| C(5)-C(4)-O(10)-Si(3) | 139.18(7) | O(4)-C(3)-C(19)-O(6) | -40.01(10) |
| C(3)-C(4)-O(10)-Si(3') | -126.11(7) | C(1)-C(3)-C(19)-O(6) | -159.23(7) |
| C(5)-C(4)-O(10)-Si(3') | 111.44(7) | C(4)-C(3)-C(19)-O(6) | 74.21(9) |
| C(38)-Si(3)-O(10)-C(4) | -35.81(13) | C(19)-O(6)-C(20)-O(7) | 4.86(13) |
| C(37)-Si(3)-O(10)-C(4) | 86.03(12) | C(19)-O(6)-C(20)- | -174.67(7) |
| C(39)-Si(3)-O(10)-C(4) | -155.06(13) | C(21) | |
| C(38)-Si(3)-O(10)- | 43.88(10) | O(7)-C(20)-C(21)- | 3.29(14) |
| Si(3') | | C(26) | |
| C(37)-Si(3)-O(10)- | 165.72(11) | O(6)-C(20)-C(21)- | -177.18(8) |
| Si(3') | | C(26) | |
| C(39)-Si(3)-O(10)- | -75.37(12) | O(7)-C(20)-C(21)- | -174.41(10) |
| Si(3') | | C(22) | |
| C(38')-Si(3')-O(10)- | 62.90(13) | O(6)-C(20)-C(21)- | 5.12(12) |
| C(4) | | C(22) | |
| C(37')-Si(3')-O(10)- | -55.36(17) | C(26)-C(21)-C(22)- | -0.42(13) |
| C(4) | | C(23) | |
| C(39')-Si(3')-O(10)- | -177.71(18) | C(20)-C(21)-C(22)- | 177.21(8) |
| C(4) | | C(23) | |
| C(38')-Si(3')-O(10)- | -53.99(11) | C(21)-C(22)-C(23)- | 0.04(14) |
| C(24) | | C(35) | |
| C(22)-C(23)-C(24)- | 0.21(16) | C(34)-C(35)-C(36)- | -1.10(17) |
| C(25) | | C(31) | |
| C(23)-C(24)-C(25)- | 0.09(17) | O(10)-Si(3)-C(39)- | 55.4(3) |
| C(26) | | C(41) | |
| C(24)-C(25)-C(26)- | -0.28(17) | | |
| C(21) | | C(38)-Si(3)-C(39)- | -60.1(3) |
| C(22)-C(21)-C(26)- | 0.53(15) | C(41) | |
| C(25) | | C(37)-Si(3)-C(39)- | 179.3(2) |
| C(20)-C(21)-C(26)- | -177.22(9) | C(41) | |
| C(25) | | O(10)-Si(3)-C(39)- | -66.3(3) |
| 0(5)-C(5)-C(27)-N(1) | -41 (4) | C(40) | |
| C(6)-C(5)-C(27)-N(1) | 77(4) | C(38)-Si(3)-C(39)- | 178.2(2) |
| C(4)-C(5)-C(27)-N(1) | -161(4) | C(40) | |
| N(2)-C(6)-C(28)-C(29) | -9.22(5) | C(37)-Si(3)-C(39)- | 57.6(3) |
| C(2)-C(6)-C(28)-C(29) | 102.09(6) | C(40) | |
| C(5)-C(6)-C(28)-C(29) | -123.32(6) | O(10)-Si(3)-C(39)- | 173.9(3) |
| O(9)-N(2)-C(29)-O(8) | 27.81(14) | C(42) | |
| C(6)-N(2)-C(29)-O(8) | 167.77(9) | C(38)-Si(3)-C(39)- | 58.5(3) |
| O(9)-N(2)-C(29)-C(28) | -150.45(7) | C(42) | |
| C(6)-N(2)-C(29)-C(28) | -10.49(6) | C(37)-Si(3)-C(39)- | -62.1(3) |
| C(6)-C(28)-C(29)-O(8) | -168.15(10) | C(42) | |
| C(6)-C(28)-C(29)-N(2) | 9.99(6) | O(10)-Si(3')-C(39')- | -166.9(4) |
| N(2)-O(9)-C(30)-C(31) | -162.46(7) | C(42') | |
| O(9)-C(30)-C(31)- | -127.97(9) | C(38')-Si(3')-C(39')- | -50.2(5) |
| C(36) | | C(42') | |
| O(9)-C(30)-C(31)- | 51.57(11) | C(37')-Si(3')-C(39')- | 67.5(5) |
| C(32) | | C(42') | |
| C(36)-C(31)-C(32)- | 1.45(15) | O(10)-Si(3')-C(39')- | -44.7(4) |
| C(33) | | C(41') | |
| C(30)-C(31)-C(32)- | -178.09(10) | C(38')-Si(3')-C(39')- | 72.0(4) |
| C(33) | | C(41') | |
| C(31)-C(32)-C(33)- | -1.30(17) | C(37')-Si(3')-C(39')- | -170.3(3) |
| C(34) | | C(41') | |
| C(32)-C(33)-C(34)- | -0.06(18) | O(10)-Si(3')-C(39')- | 74.8(4) |
| C(35) | | C(40') | |
| C(33)-C(34)-C(35)- | 1.26(18) | C(38')-Si(3')-C(39')- | -168.5(4) |
| C(36) | | C(40') | |
| C(32)-C(31)-C(36)- | -0.26(15) | C(37')-Si(3')-C(39')- | -50.8(5) |
| C(35) | | C(40') | |
| C(30)-C(31)-C(36)- | 179.28(10) | | |

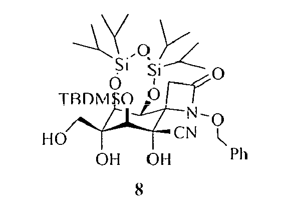

***R_{f}=*** 0.16 (TLC, hexane/AcOEt 3:1); yield, 10%; white solid;
**¹H-NMR** (300 MHz CDCl₃): δ 7.46- 7.43 (2H, m, Ph), 7.39-7.35 (3H, m, Ph), 5.17 (1H, *part A syst. AB,* J = 10.7 Hz, OCH₂Ph), 5.12 (1H, *part B syst. AB,* J = 10.7 Hz, OCH₂Ph), 4.86 (1 H, br. s, OH), 4.81 (1H, d, J = 4.8 Hz), 4.36 (1 H, d, J = 4.8 Hz), 4.14 (2H, s, CH₂OH), 4.06 (1 H, br. s, OH), 3.98 (1 H, dd, J = 10.3, 5.3 Hz), 3.66 (1H, dd, J = 10.3, 3.4 Hz), 3.30 (1H, *partA syst. AB,* J = 14.8 Hz, H-3), 2.87 *(1H, part B syst. AB,* J = 14.8 Hz, H-3'), 2.10 (1H, br. s), 1.13-1.01, (28H, m, TIPDS), 0.93 (9H, s, C(CH₃)₃), 0.29 (3H, s, SiCH₃), 0.22 (3H, s, SiCH₃);
**¹³C-NMR** (75 MHz, CDCl₃):167.1, 135.5, 129.9, 129.0, 128.9, 128.6, 128.4, 117.9, 79.4, 78.7, 77.2, 75.1, 73.6, 68.0, 65.1, 63.7, 38.5, 26.4, 17.8, 17.5, 17.4, 17.3, 17.2, 17.1,16.9,13.9,13.0,12.9;
**LMRS(API-ES⁺):** m/z 737 (M+H)⁺, 738 (M+2H)⁺, 739 (M+3H)⁺, 759 (M+Na)⁺, 760 (M+Na+H)⁺; 783 (M+2Na+H)⁺; 1497 (2M+Na+2H)⁺.

### Example 5: compounds of formula (Ib) and Epimerization

### Preparation of rac-(4S,SR,6S,7R,8S,9S)-1-benzyloxy-7-(benzyloxymethyl)-6-(tert-butyldimethylsililoxi)-5,8,9-trihydroxy-8,9-O-(1,1,3,3-tetraisopropyldisiloxane-1,3-diyl)-1-azaspiro[3.5]non-2-ona-5,7-carbolactone (10) and rac-(4R,5R,6R,7S,8S,9S)-1-benzyloxy-7-(benzoyloxymethyl)-6-(tert-butyldimethylsilyloxy)-5,7-O-di-tert-butylsiloxane-5-cyano-5,7,8,9-tetrahydroxy-8,9-O-(1,1,3,3-tetraisopropyldisiloxane-1,3-diyl)-1-azaspiro[3.5]nonan-2-one (11)

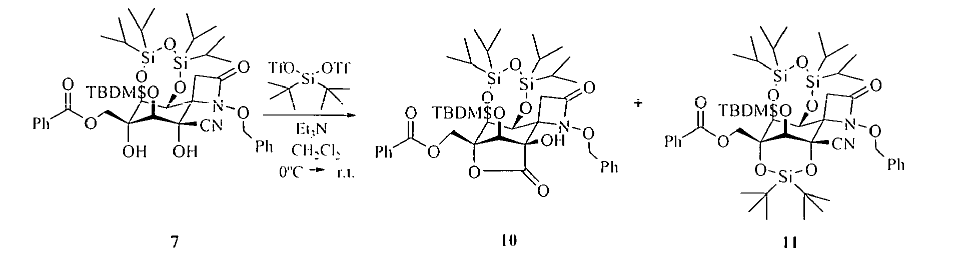

To a stirred solution of *rac*-(4*R*,5*R*,6*R*,7*S*,8*S*,9*S*)-1-benzyloxy-7-(benzoyloxy methyl)-6-(*tert*-butyldimethylsilyloxy)-5-cyano-5,7,8,9-tetrahydroxy -8,9-*O-*(1,1,3,3-tetraisopropyldisiloxane-1,3-diyl)-1-azaspiro[3.5]nonan-2-one (**7**) (50 mg, 0.060 mmol) in CH₂Cl₂ (2 ml) at 0° C was added triethylamine (20µl, 0.143 mmol) and di-*tert*-butylsilyl bis(trifluomethane)sulfonate (22µl, 0.065 mmol). The resulting mixture was stirred at room temperature for 17 hours. The reaction was quenched at 0° C with Na₂PO₄ (0.1 M buffer, 3 ml) and the mixture extracted with AcOEt (3 x 5 ml). The combined organic extracts were dried over Na₂SO₄, filtered and concentrated under reduced pressure. The residue was purified by silica gel column chromatography (hexane/AcOEt, 8:1) to give rac-(4*S*,*5R*,6*S*,7*R*,8*S*,9*S*)-1-benzyloxy-7-(benzyloxymethyl)-6-(*tert*-butyldimethylsililoxi)-5,8,9-trihydroxy-8,9-*O*-(1,1,3,3-tetraisopropyldisiloxane-1,3-diyl)-1-azaspiro[3.5]non-2-ona-5,7-carbolactone (**10**) (30 mg, 33%) as a white solid (24 mg, 48%) and *rac-(4R,5R,6R,7S,8S,9S)-*1-benzyloxy-7-(benzoyloxymethyl)-6-(*tert*-butyldimethylsilyloxy)-5,7-*O*-di-*tert-*butylsiloxane-5-cyano-5,7,8,9-tetrahydroxy-8,9-*O*-(1,1,3,3-tetraisopropyldisiloxane-1,3-diyl)-1-azaspiro[3.5]nonan-2-one **(11)** (17 mg, 29%). 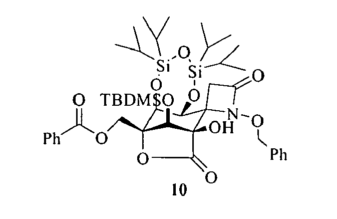 ***R_{f}=*** 0.14 (TLC, hexane/AcOEt 4:1); **yield,** 48%; white solid;
**¹H-NMR** (300 MHz CDCl₃): δ 8.02 (2H, dm, J = 7.1 Hz, Bz), 7.61 (1H, t, J = 7.3 Hz, Bz), 7.47 (2H, dd, J = 7.3 Hz, Bz), 7.43-7.25 (5H, m, Ph), 5.16 (1H, *part A syst. AB,* J = 9.8 Hz, OCH₂Ph), 5.08 (1H, *part B* syst. *AB,* J = 9.8 Hz, OCH₂Ph), 4.69 (1H; *part A syst. AB, J* = 12.4 Hz, H-4), 4.67 (1H, d, J = 3.4 Hz, H-9), 4.57 (1H, *part B syst. AB*,, J = 12.4, Hz, H-10'), 4.56 (1H, d, J = 3.4 Hz, H-8), 4.49 (1H, s, H-6), 3.31 (1H, br.s, OH), 3.14 (1H, *part A syst. AB, J* = 14.4 Hz, H-3), 3.03 (1H*, part B syst*. *AB,* J = 14.4 Hz, H-3'), 1.16-0.80 (28H, m, TIPDS), 0.90 (9H, s, C(CH₃)₃), 0.16 (3H, s, SiCH₃), 0.12 (3H, s, SiCH₃);
**IR** (film): ν 3435, 3062, 3032, 2962, 2925, 2867, 1769, 1729, 1663, 1601, 1465, 1387, 1313, 1262,1099, 1015, 885, 801, 736, 696 cm⁻¹ ;
**LMRS(API-ES⁺):** m/z 841 (M)⁺, 842 (M+H)⁺, 843 (M+2H)⁺, 864 (M+Na)⁺, 1705 (2M+Na)⁺.
**X-ray Diffaction of *rac*-(4*S*,5*R*,6*S*,7*R*,8*S*,9*S*)-1-benzyloxy-7-(benzyloxymethyl)-6-(*tert-*butyldimethylsililoxi)-5,8,9-trihydroxy-8,9-*O*-(1,1,3,3-tetraisopropyldisiloxane-1,3-diyl)-1-azaspiro[3.5]non-2-ona-5,7-carbolactone (10)**

A solution *rac*-(4*S*,5*R*,6*S*,7*R*,8*S*,9*S*)-1-benzyloxy-7-(benzyloxymethyl)-6-(*tert-*butyldimethylsililoxi)-5,8,9-trihydroxy-8,9-*O*-(1,1,3,3-tetraisopropyldisiloxane-1,3-diyl)-1-azaspiro[3.5]non-2-ona-5,7-carbolactone (**10**) in AcOEt (24 mM, 0.5 ml disol.) was crystallized by hexane diffusion.

The crystal was placed on a *Mar345* diffractometer equipped with a *image-plate* detector. The determination of unit cell parameters and data collections were performed with Mo*Ka* radiation using a graphite monochromator. The program used for evaluation was *WINGX,* the intensities were corrected using Lorente-polarization factor. The structure was solved by direct methods using the *SHELXL-97* program. Final refinement was performed by full-matrix least-squares methods. Crystallographic data and experimental details for structural analyses are summarized in Table 4, whereas positional parameters and atomic coordinates are given in Table 5. Figure 2 is a representation of the molecular structure obtained using X-rays diffraction.

**Table 4. Crystal data and structure**

| | |
|---|---|
| Empirical formula | C₄₂H₆₃NO₁₁Si₃ |
| Formula weight | 842.20 |
| Temperature | 293(2) K |
| Wavelength (Å) | 0.71073 |
| Crystal system, space group | Monoclinic, P21/a |
| Unit cell dimensions | a = 15.706(6) Å |
| | b=16.444(5) |
| | c = 19.902(6) |
| | β = 107.62(2)" |
| Volume | 4899(3) Å³ |
| Z, Calculated density | 4, 1.142 Mg/m³ |
| µ (mm⁻¹) | 0.149 |
| F(000) | 1808 |
| Crystal size | 0.3 x 0.2 x 0.2 mm |
| θ range for data collection | 3.44 to 32.98° |
| Reflections collected / unique | 42348 / 12584 [R(int) = 0.0434] |
| Goodness-of-fit on F² | 1.070 |
| Final R indices [I>2sigma(I)] | R1 = 0.0569, wR2 = 0.1856 |
| R indices (all data) | R1 = 0.0875, wR2 = 0.2066 |

**Table 5. Atomic coordinates (x 10⁴) and equivalent isotropic displacement parameters (Å² x 10³). U(eq) is defined as one third of the trace of the orthogonalized Uᵢⱼ tensor.**

| | x | y | z | U(eq) |
|---|---|---|---|---|
| Si(2) | 1874(1) | 4420(1) | 4123(1) | 61 (1) |
| Si(3) | -178(1) | 7035(1) | 2366(1) | 62(1) |
| Si(1) | 2977 (1) | 3674 (1) | 3284 (1) | 64 (1) |
| O(1) | 2489(1) | 4341 (1) | 2669(1) | 58 (1) |
| O(2) | 1275(1) | 4851 (1) | 3370(1) | 51 (1) |
| 0(3) | 2622(1) | 3841 (1) | 3964 (1) | 65 (1) |
| 0(5) | -1521(1) | 4668 (1) | 2257(1) | 66(1) |
| 0(6) | -2013(1) | 5059(1) | 3161(1) | 95 (1) |
| O(7) | 330(1) | 6137 (1) | 2398(1) | 53(1) |
| 0(8) | 2233(1) | 4286(1) | 1063(1) | 57 (1) |
| O(9) | 3384(1) | 5749(1) | 1643(1) | 78 (1) |
| O(10) | -256(1) | 4111(1) | 1714(1) | 51 (1) |
| O(11) | 293 (1) | 5861(1) | 864 (1) | 48 (1) |
| 0(12) | -255(1) | 4172(1) | 552(1) | 75(1) |
| N | 1989(1) | 5026(1) | 1289(1) | 50(1) |
| C(1) | 1573(1) | 4379(1) | 2283(1) | 47 (1) |
| C(2) | 950 (1) | 4386(1) | 2751(1) | 48 (1) |
| C(3) | -3(1) | 4664 (1) | 2319(1) | 47 (1) |
| C(4) | -51(1) | 5485(1) | 1945(1) | 45 (1) |
| C(5) | 439 (1) | 5255(1) | 1402 (1) | 42 (1) |
| C(6) | 1443 (1) | 5102(1) | 1782(1) | 42 (1) |
| C(7) | 4178(2) | 3890(2) | 3527(2) | 91 (1) |
| C(8) | 4734 (2) | 3465(3) | 4181 (2) | 105 (1) |
| C(9) | 4610(2) | 3599(3) | 2928(2) | 108 (1) |
| C(10) | 2727 (2) | 2620(2) | 2978(2) | 91 (1) |
| C(11) | 2843(2) | 2489(2) | 2268(2) | 99(1) |
| C(12) | 1885 (2) | 2332(2) | 2918 (2) | 104 (1) |
| C(13) | 1096(2) | 3743(2) | 4437 (2) | 87 (1) |
| C(14) | 1619(2) | 3093(2) | 4985(2) | 93(1) |
| C(15) | 458 (2) | 4222(2) | 4698 (2) | 92 (1) |
| C(16) | 2388(2) | 5275(2) | 4696(2) | 87 (1) |
| C(17) | 2949(2) | 5790(2) | 4364 (2) | 97 (1) |
| C(18) | 2933(2) | 4999(2) | 5443(2) | 97 (1) |
| C(19) | -629(2) | 9577(2) | 2731(2) | 78 (1) |
| C(20) | -2170(2) | 4866(2) | 2559(2) | 72 (1) |
| C(21) | -3060(2) | 4856(2) | 2045(2) | 82 (1) |
| C(22) | -3173(2) | 4619(2) | 1377 (2) | 95(1) |
| C(23) | -4072(2) | 4552(2) | 927 (2) | 103(1) |
| C(24) | -4732(2) | 4857(2) | 1290 (2) | 102(1) |
| C(25) | -4594 (2) | 5114 (2) | 1908 (2) | 96(1) |
| C(26) | -3741(2) | 5070 (2) | 2282(2) | 92 (1) |
| C(27) | 326(2) | 7466(2) | 3271 (2) | 87 (1) |
| C(28) | 270(2) | 6902(2) | 3821(2) | 97 (1) |
| C(29) | 1339(2) | 7627 (2) | 3313(2) | 97 (1) |
| C(30) | -78 (2) | 8312(2) | 3316(2) | 95(1) |
| C(31) | -1413(2) | 6949(2) | 2191(2) | 90(1) |
| C(32) | -1 (2) | 7646(2) | 1632 (2) | 89(1) |
| C (33) | 1919(1) | 4264 (1) | 295(1) | 79(1) |
| C(34) | 2339(1) | 3530(1) | 82 (1) | 66(1) |
| C(35) | 3078 (1) | 3630(1) | -156(1) | 98 (1) |
| C(36) | 3444 (2) | 2958 (3) | -400(2) | 109(1) |
| C(37) | 3076(2) | 2206(2) | -374 (2) | 96(1) |
| C(38) | 2359(3) | 2108 (2) | -152 (2) | 103(1) |
| C(39) | 1982 (2) | 2775(2) | 82 (2) | 97 (1) |
| C(40) | 2614 (1) | 5600(1) | 1653 (1) | 57 (1) |
| C(41) | 2019(1) | 5877(1) | 2069(1) | 52 (1) |
| C(42) | -67 (1) | 4469(1) | 1154(1) | 47 (1) |

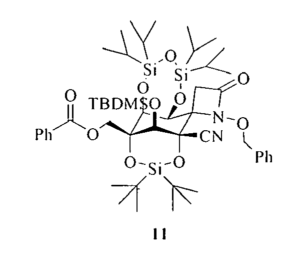

***R_{f}* =** 0.59 (TLC, hexane/AcOEt 4:1); yield, 29%;
**¹H-NMR** (300 MHz CDCl₃): δ 8.05-8.02 (2H, m, Bz), 7.62-7.59 (1H, m, Bz), 7.49-7.43 (2H, m, Bz), 7.39-7.29 (5H, m, Ph), 5.28 (1H, d, J = 4.8 Hz, H-9), 5.17 (1H, *part A syst. AB,* J = 11.2 Hz, OCH₂Ph), 5.06 (1H, *part B syst. AB,* J = 11.2 Hz, OCH₂Ph), 4.88 (1H, br. s, H-6), 4.76 (1H, *part A syst. AB,* J = 11.6 Hz, H-10), 4.64 (1H, *part B syst. AB,* J = 11.6Hz,H-10'), 4.52 (1H, d, J=4.8Hz, H-8), 3.26 (1H, *part A syst. AB,* J = 14.6 Hz, H-3), 2.88 (1H, *part B syst. AB,* J = 14.6 Hz, H-3'), 1.16-0.94, (56H, m, TIPDS, TBDS), 0.36 (3H, s, SiCH₃), 0.28 (3H, s, SiCH₃););
**¹³C**-**NMR** (75 MHz, CDCl₃): δ 167.2, 166.5, 135.5, 133.3, 129.8, 129.5, 128.4, 128.2, 128.1, 124.8, 118.1, 79.9, 78.5, 77.5, 77.2, 74.6, 73.4, 68.0, 66.6, 64.6, 39.0, 29.7, 28.5, 27.7, 27.6, 27.5, 26.8, 26.7, 26.3, 22.1, 21.4, 20.5, 20.4, 20.2, 18.7, 17.8, 17.4, 17.24, 17.22, 17.19, 17.13, 16.9, 14.9, 14.2, 13.2, 12.6, 12.2, 1.0;
**LMRS (API-ES⁺)**: m/z 981 (M)⁺, 1004 (M+Na)⁺.

### Example 6

### Preparation of rac-(1'R,4R,5S,6S,7S,8R,9S)-1-Benzylloxy-8,10-(cyanophenylmethylendioxy)-5,6,7,9-tetrahydroxy-7-methyl-5,6-O-(1,1,3,3-tetraisopropyldisyloxane-1,3-diyl)-1-azaspiro[3.5]nonan-2one (12)

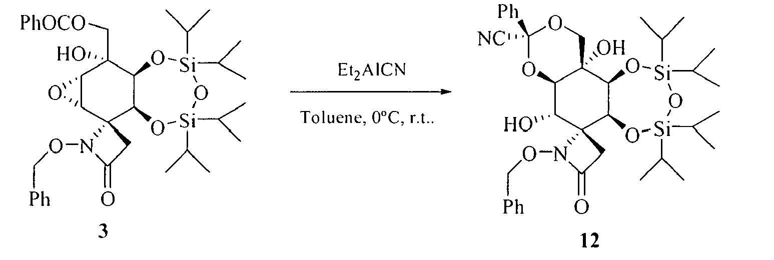

To a solution of *rac*-(4*R*,5*S*,6*S*,7*S*,8*S*,9*S*)-1-benzylloxy-7-(benzoyloxymethyl)-8,9-epoxi-5,6,7-trihydroxy-5,6-*O*-(1,1,3,3-tetraisopropyldisylixane-1,3-diyl)-1-azaspiro[3.5]nonan-2-one **(3)** (420 mg, 0.614 mmol, 1 eq) in toluene (5 ml) was added, at 0°C, diethylaluminium cyanide (0.92 ml, disol. 1.0 M in toluene, 1.5 eq). The reaction mixture was stirred for 17 hours at room temperature, after which, a mixture of potasium sodium 10% de tartrate aqueous solution-EtOAc (1:1, 4 ml). The mixture was then stirred 10 minutes, the phases separated and the aqueous phase extracted with EtOAc (3 x 3 ml). The organic phase was dried with anhydrous Na₂SO₄, filtrated and the organic solvent evaporated under reduced pressure to yield 422 mg (97 % yield before purification) of the title compound. The residue was purified by chromatographic column (hexane/AcOEt, 4:1) to yield *rac*-(1'*R*,4*R*,5*S*,6*S*,7*S*,8*R*,9*S*)-1-Benzyloxy-8,10-(cyanophenylmethylendioxy)-5,6,7,9-tetrahydroxy-7-methyl-5,6-*O*-(1,1,3,3-tetraisopropyldysiloxane-1,3-diyl)-1-azaspiro[3.5]nonan-2-one (**12**) (55 % yield) as a white solid.

### rac-(1'R,4R,5S,6S,7S,8R,9S)-1-Benzyloxy-8,10-(cyanophenylmethylendioxy)-5,6,7,9-tetrahydroxy-7-methyl-5,6-O-(1,1,3,3-tetraisopropyldysiloxane-1,3-diyl)-1-azaspiro [3.5]nonan-2-one (12)

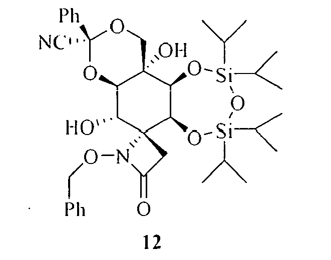

***R_{f}=*** 0.35 (TLC, Hex: AcOEt 3:1). **Yield** 98%; white solid.
**¹H-NMR** (200 MHz, CDCl₃): δ 7.56- 7.51 (2H, m, Ph), 7.46- 7.31 (8H, m, Ph), 5.19 (1H, part. A syst.AB, J = 10.4 Hz), 5.08 (1H, part.B *syst.* AB, J = 10.4 Hz), 5.01 (1H, d, J = 3.7 Hz, H-5) 4.52 (1H, dd, J = 3.7, 1.5 Hz, H-6), 4.41 (1H, dd, 2.7, 1.6 Hz, H-8), 4.32 (1H, t, J = 3.3, 2.9 Hz, H-9), 4.22 (1H, part. A *syst.* AB, J =12.1 Hz, H-11), 4.11 (1H, part. B *syst.*AB, J = 12.1 Hz, H-11), 3.93 (3 H, s, OH(7)), 3.54 (1H, br. s, OH(9)), 3.11 (1H, part. A syst. AB, J = 14.28 Hz, H-3), 2.34 (1H, part.B *syst.*AB*,* J = 14.28 Hz, H-3) 1.10- 1.00 (28H, m, TIPDS).
**¹³C-NMR** (75MHz, CDCl₃) δ 164.7, 135.6, 134.4, 130.3, 129.4, 129.2, 128.9, 128.3, 125.6, 114.5 (CN), 95.2, 79.2, 78.1, 76.7, 76.4, 70.7, 67.7, 65.8, 65.6, 37.3, 17.8, 17.6, 17.5, 17.2, 17.1, 16.8, 16.7, 16.6, 14.2, 13.1, 13.0.
**IR** (KBr): ν 3451, 2946, 2889, 2862, 2218, 1748, 1631, 1462, 1278, 1248, 1154, 1099 1055, 922, 883, 855, 804, 744, 695 cm⁻¹.
**LMRS** (API-ES⁺): m/z 1444 (2M+Na)⁺, 733 (M+Na)⁺, 711 (M+H)⁺, 684 (M-26)⁺.
**LMRS** (EI): m/z 710 (M⁺,0.2), 683 (0.5), 588 (1), 561 (1), 532 (1), 518 (1), 439 (1), 399 (2), 365 (2), 339 (7), 289 (5), 261 (6), 191 (4), 163 (4), 147 (6), 135 (11), 119 (8), 105 (98), 91 (100), 77 (19).

### Preparation of rac-(1'R,4S,5S,6S,7S,8R)-1-benzyloxy-8,10-(cyanofenylmethylendioxy) -5,6,7-trihydroxy-7-methyl-5,6-O-(1,1,3,3-tetraisopropyldisiloxane-1,3-diyl)-1-azaespiro[3.5]nonan-2,9-dione (14) and rac-(1'R,4S,5S,6S,7S,8R)-1-benciloxy-8,10-(cyanofenylmethylendioxy)-5,6,7,9,9-pentahydroxy-7-methyl-5,6-O-(1,1,3,3-tetraisopropyldisiloxane-1,3-diyl)-1-azaespiro[3.5]nonan-2one (15)

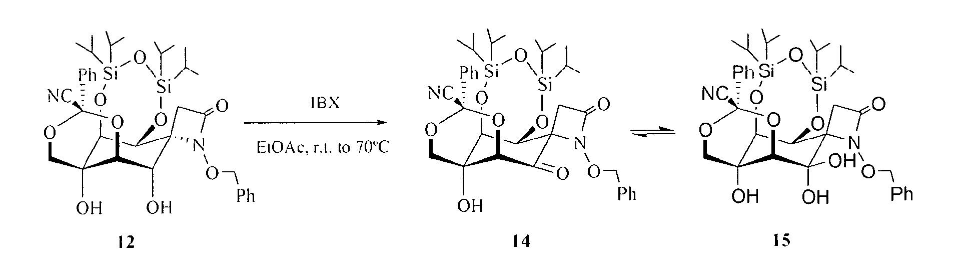

To a stirred solution of *rac-*(1*'R,4*R*,5*S*,6*S*,7*S*,8*R*,9*S*)-*1-benciloxy-8,10-(cyanofenylmethylendioxy)-5,6,7,9-tetrahydroxy-7-methyl-5,6-*O*-(1,1,3,3-tetraisopropyldisiloxane-1,3-diyl)-1-azaespiro[3.5]nonan-2one (**12**) (200 mg, 0.242 mmol, 1 eq) in AcOEt (8 ml), at room temperature, was added IBX (237 mg, 3 eq). The resulting mixture was heated at 70°C for 3 hours. After this time, the mixture was filtered through Celite in vacuum and concentrated under reduced pressure. To the result solid in AcOEt (8 ml), was added, at room teperature IBX (237 mg, 3 eq) and the mixture was heated at 70°C. After 3 hours, the mixture was filtered again through Celite in vacuum and concentrated under reduced pressure.suspension. To the result solid in AcOEt (8 ml), was added, at room temperature IBX (237 mg, 3 eq) and the mixture was heated at 70°C for 3 hours. After this time, the mixture was filtered through Celite in vacuum and concentrated under reduced pressure. To the solid product was added AcOEt (10 ml) and the mixture formed was washed with water (2x 10 ml). The layers were separated. The organic layer was dried over Na₂SO₄, filtered and concentrated under reduced pressure. To the solid was added diethyl ether (5 ml) and was sonicated by means of an ultrasound bath for 5 minutes. After this time, the solvent was evaporationed under reduced pressure. To the solid was added toluene (5 ml) and was sonicated by means of an ultrasound bath for 5 minutes. After this time, the solvent was evaporationed under reduced pressure. To the solid was added, diethyl ether (5 ml) and was sonicated by means of an ultrasound bath for 5 minutes. After this time, the solvent was evaporationed under reduced pressure. To the solid was added toluene (5 ml) and was sonicated by means of an ultrasound bath for 5 minutes. After this time, the solvent was evaporationed under reduced pressure. To the solid was added diethyl ether (5 ml) and was sonicated by means of an ultrasound bath for 5 minutes. After this time, the solvent was evaporationed under reduced pressure to give a mixture [10:1] of *rac-*(1'*R*,4*S*,5*S*,6*S*,7*S*,8*R*)-1-benzyloxy-8,10-(cyanofenylmethylendioxy)-5,6,7-trihydroxy-7-methyl-5,6-*O*-(1,1,3,3-tetraisopropyldisiloxane-1,3-diyl)-1-azaespiro[3.5]nonan-2,9-dione **(14)** and *rac*-(1'*R*,4*S*,5*S*,6*S*,7*S*,8*R*,)-1-benciloxy-8,10-(cyanofenylmethylendioxy)-5,6,7,9,9-pentahydroxy-7-methyl-5,6-*O*-(1,1,3,3-tetraisopropyldisiloxane-1,3-diyl)-1-azaespiro[3.5]nonan-2one **(15)** (179 mg, yield 90% ).

### rac-(1'R,4S,5S,6S,7S,8R)-1-benzyloxy-8,10-(cyanofenylmethylendioxy)-5,6,7-trihydroxy-7-methyl-5,6-O-(1,1,3,3-tetraisopropyldisiloxane-1,3-diyl)-1-azaespiro[3.5]nonan-2,9-dione (14)

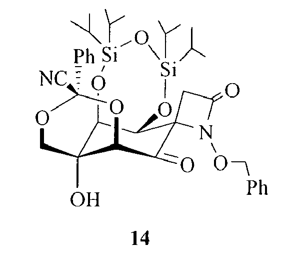

**¹H- NMR** (300 MHz, CDCl₃): δ 7.60- 7.55 (2H, m, Ph), 7.45- 7.30 (8H, m, Ph), 5.16 (2H, s, OCH₂Ph), 4.78 (1H, d, J = 3.7 Hz, H-5) 4.62 (1H, d, J = 1.5 Hz, H-8), 4.57 (1H, dd, 3.7, 1.5 Hz, H-6), 4.38 (1H, part. A syst. AB, J =12.1 Hz, H-10), 4.16 (1H, part. B syst.AB, J = 12.1 Hz, H-10'), 3.36 (1H, part. A syst. AB, J = 13.8 Hz, H-3), 2.64 (1H, part.B syst.AB, J = 13.8 Hz, H-3') 1.13- 1.01 (28H, m, TIPDS).
**¹³C- NMR** (75 MHZ, CDCl₃): δ 198.5, 163.9, 135.4, 134.8, 130.6, 129.2,128.8, 128.4,
128.3, 125.5, 114.1, 94.7, 81.2, 78.7, 76.5, 73.0, 70.5, 68.7, 65.2, 38.6, 17.8, 17.4, 17.3, 17.0, 16.9, 16.8, 16.7, 16.6.
**LMRS(API-ES⁺):** m/z 754 (M+2Na)⁺, 731 (M+Na)⁺, 708 M⁺.

### rac-(1'R,4S,5S,6S,7S,8R,9S)-1-benciloxy-8,10-(cyanofenylmethylendioxy)-5,6,7,9,9-pentahydroxy-7-methyl-5,6-O-(1,1,3,3-tetraisopropyldisiloxane-1,3-diyl)-1-azaespiro[3.5]nonan-2one (15)

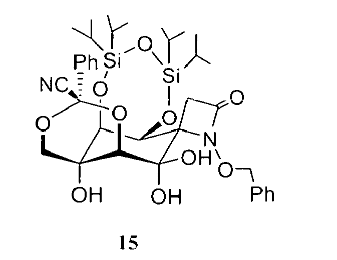

**¹H-NMR** (300 MHz, CDCl₃): δ 7.60- 7.55 (2H, m, Ph), 7.45- 7.30 (8H, m, Ph), 5.20 (1H, part.A syst. AB, J =10.6 Hz, OCH₂Ph), 5.03 (1H, part.B syst. AB, J = 10.6 Hz, OCH₂Ph), 4.84 (1 H, d, J =3.5 Hz, H-5) 4.54 (1 H, brs), 4.46 (1 H, dd, J = 3.5, 1.3 Hz, H-6), 4.26 (1H, part.A syst.AB, J = 12.3 Hz, H-10), 4.19 (1H, d, J = 1.3 Hz, H-8), 4.11 (1H, part.B syst.AB, J = 12.3 Hz, H-10'), 2.98 (1H, part. A syst. AB, J =13.9 Hz, H-3), 2.76 (1H, part. B syst.AB, J = 13.9 Hz, H-3'), 1.13- 1.01 (28H, m, TIPDS).
**LMRS(API-ES⁺):** m/z 754 (M+2Na)⁺, 731 (M+Na)⁺, 708 M⁺.

### Preparation of rac-(4R,5S,6S,7S,8S,9S)-1-benzyloxy-6,10-cyanophenylmethylendioxy -5,7,8,9-tetrahydroxy-7-methyl-8,9-O-(1,1,3,3-tetraisopropyldisiloxane-1,3-diyl)-1-azaspiro[3.5]non-2-ona (16)

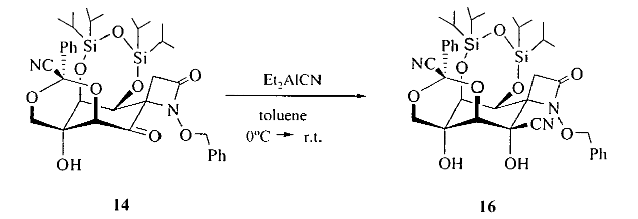

To a solution of *rac*-(4*R*,5*R*, *6S, 7R,8S,9S)-*1-benzyloxy-5-carbonyl-6,10-cyanophenylmethylendioxy-5,7,8-trihydroxy-7-methyl-8,9-*O*-(1,1,3,3-tetraisopropyldi siloxane-1,3-diyl)-1-azaspiro[3.5]non-2-one (**14**) (90 mg, 0.130 mmol) in toluene (1 ml) was added, at 0 °C with vigorous stirring, a solution of diethylaluminium cyanide in toluene (1.0 M, 0.20 ml, 0.200 mmol) and the mixture was stirring to room temperature. After 14 hours, a mixture 10% aquous potassium-sodium tartrate solution-AcOEt (1:1, ml) was added. The layers were separated and aqueous phase was extracted with AcOEt (3 x 3 ml). The combined organic extracts were dried over Na₂SO₄, filtered and concentrated under reduced pressure to give *rac*-(4*R*,5*S*,6*S*,7*S*,8*S*,9*S*)-1-benzyloxy-6,10-cyanophenylmethylendioxy -5,7,8,9-tetrahydroxy-7-methyl-8;9-*O*-(1,1,3,3-tetraisopropyl disiloxane-1,3-diyl)-1-azaspiro[3.5]non-2-ona (**16**) (70 mg, 73%) as a white solid. 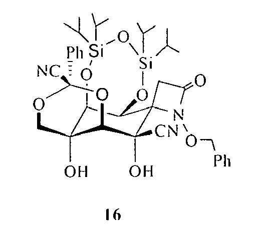 ***R_{f}**=* 0.25 (TLC, hexane/AcOEt 3:1); yield, 73%; white solid.
**¹H-NMR** (300 MHz, CDCl₃): δ 7.55- 7.33 (10H, m, Ph), 5.18 (1H, *part A syst. AB,* J = 11.1 Hz, OCH₂Ph), 5.01 (1H, *part B syst.* AB, J = 11. Hz, OCH₂Ph), 4.82 (1H, d, J = 3.6 Hz, H-9), 4.48 (2H, br. s, H-8 y H-6), 4.24 (1H, *part A syst. AB,* J =12.2 Hz, H-10), 4.09 (1H, *part B syst. AB,* J = 12.2 Hz, H-10'), 3.23 (1 H, *part A syst. AB,* J = 14.29 Hz, H-3), 2.76 (1H, *part B syst. AB,* J = 14.29 Hz, H-3') 1.10- 1.00 (28H, m, TIPDS);
**¹³C -NMR** (75 MHz, CDCl₃): δ 166.1, 134.8, 134.7, 130.6, 129.3, 129.2, 129.0, 128.9, 128.7, 128.6, 128.2, 125.4, 125.3, 115.4, 113.9, 95.1, 79.2, 77.5, 77.2, 70.2, 67.3, 65.7, 65.1, 38.2, 29.7, 17.7, 17.5, 17.4, 17.2, 17.0, 16.9, 16.8, 16.6, 16.5, 14.0, 13.4, 13.1, 12.9, 1.0;
**LMRS (API-ES⁺):** m/z 758 (M+Na)⁺, 759 (M+Na+H)⁺, 737 (M+2H)⁺, 736 (M+H)⁺.
**IR** (film): ν 3435.8, 3065, 3034.2, 2959, 2947,2, 2866.0, 2344, 2244, 1760.9, 1624.0, 1453.2, 1261.7, 1105.2, 1008.9, 884.6, 800.7, 696.7

### Example 9: General Formula I

### Preparation of rac-(4S,5R,6S,7R,8S,9S)-1-benzyloxy-7-(benzyloxymethyl)-6-(tert-butyldimethylsililoxi)-5,8,9-trihydroxy-8,9-O-(1,1,3,3-tetraisopropyldisiloxane-1,3-diyl)-1-azaspiro[3.5]non-2-ona-5,7-carbolactone (10)

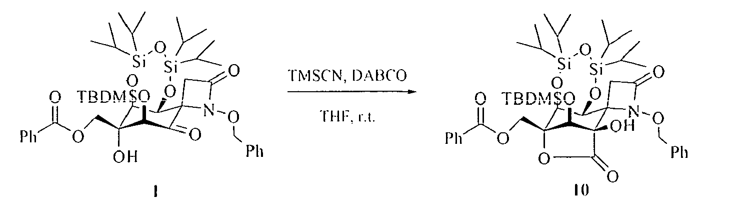

To a stirred solution of *rac*-(4*S*,6*S*,7*R*,8*S*,9*S*)-1-bencyloxy-7-benzoiloximetil-6*-tert-*butildimetilsililoxi- 7,8,9-trihydroxy-8,9-*O*-( 1,1,3,3-tetraisopropildisiloxano-1,3-diyl)-1-azaespiro[3.5]nonano-2,5-diona (**1**) (92 mg, 0.113 mmol) and trimethylsilyl cyanide (29 µl, 0.216 mmol) in THF (1 ml) at room temperature was slowly added DABCO (1 mg, 0.01 1 mmol). The mixture was stirred at room temperature for 15 h and then concentrated under reduced pressure. The residue was purified by silica gel column chromatography (hexane-AcOEt, 10:1) to give rac-(4*S*,*5R*,6*S*,7*R*,8*S*,9*S*)-1-benzyloxy-7-(benzyloxymethyl)-6-(*tert*-butyldimethylsililoxi)-5,8,9-trihydroxy-8,9-*O*-(1,1,3,3-tetraisopropyldisiloxane-1,3-diyl)-1-azaspiro[3.5]non-2-ona-5,7-carbolactone (**10**) (30 mg, 33%) as a white solid. 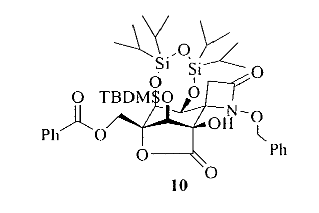 ***R_{f} =*** 0.30 (TLC, hexane/AcOEt, 1:1); **yield**, 33%; white solid;
**¹H-NMR** (300 MHz, CDCl₃): δ 8.02 (2H, dm, J = 7.1 Hz, Bz), 7.61 (1H, t, J = 7.3 Hz, Bz), 7.47 (2H, dd, J = 7.3 Hz, Bz), 7.43-7.25 (5H, m, Ph), 5.16 (1H*, part A syst. AB,* J = 9.8 Hz, OCH₂Ph), 5.08 (1H, *part B syst. AB,* J = 9.8 Hz, OCH₂Ph), 4.69 (1H, *part A syst. AB, J* = 12.4 Hz, H-4), 4.67 (1H, d, J = 3.4 Hz, H-9), 4.57 (1H*, part B syst. AB,,* J = 12.4, Hz, H-10'), 4.56 (1H, d, J = 3.4 Hz, H-8), 4.49 (1H, s, H-6), 3.31 (1H, br.s, OH), 3.14 (1H, *part A syst. AB,* J = 14.4 Hz, H-3), 3.03 (1H, *part B syst. AB,* J = 14.4 Hz, H-3'), 1.16-0.80 (28H, m, TIPDS), 0.90 (9H, s, C(CH₃)₃), 0.16 (3H, s, SiCH₃), 0.12 (3H, s, SiCH₃);
**IR** (film): ν 3435, 3062, 3032, 2962, 2925, 2867, 1769, 1729, 1663, 1601, 1465, 1387, 1313, 1262, 1099, 1015, 885, 801, 736, 696 cm⁻¹;
**LMRS (API-ES⁺):** m/z 1705 (2M+Na)⁺, 864 (M+Na)⁺, 841 (M)⁺.

## Claims

1. A compound of formula (I) 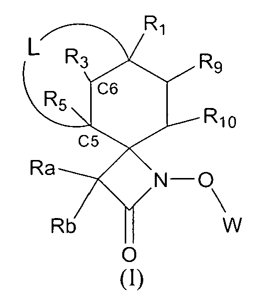 wherein
R₁ is selected from hydrogen, cyano, substituted or unsubstituted alkyl, alkyl-OPr, substituted or unsubstituted alkenyl, substituted or unsubstituted alkynyl and NReRf, wherein Re and Rf are each independently selected from H, OH, OPr, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy, substituted or unsubstituted amino and halogen; and
R₃ is selected from hydrogen, OH, OPr or =O; or
R₁ and R₃ together form a group selected from -CH₂-O-Pr-O- and -CH₂-O-C(R₁₁R₁₂)-O-, the oxygen terminus being attached to the C6 carbon atom; wherein R₁₁ is selected from the group consisting of substituted or unsubstituted alkyl, substituted or unsubstituted cycloalkyl, substituted or unsubstituted alkenyl, substituted or unsubstituted aryl, substituted or unsubstituted aralkyl, substituted or unsubstituted alkoxy, substituted or unsubstituted aryloxy,; and
R₁₂ is selected from the group consisting of cyano, substituted or unsubstituted alkyl and aziridine;
R₉ and R₁₀ are each independently selected from hydrogen, OH, OPr and =O;
R₅ is selected from the group consisting of hydrogen, -CN,-OH, OPr and a carbonyl derivative of formula -C(=O)Rg, wherein Rg is a selected form the group consisting of hydrogen, substituted or unsubstituted alkoxy, substituted or unsubstituted aryloxy and substituted or unsubstituted amine;
L is selected from the group consisting of
• -O-C(=X)-, wherein X is selected from NPrn, NH or O, and the carbon atom supporting X is attached to C5; and
• Z, wherein Z is selected from the group consisting of -O-C(R₆R₇)-O-, -O-Si(R₆R₇)-O- and -O-Si(R₆R₇)-O-Si(R₆R₇)-O-, wherein each of R₆ and R₇ are independently selected from the group consisting of hydrogen, substituted or unsubstituted alkyl, substituted or unsubstituted cycloalkyl, substituted or unsubstituted alkenyl, substituted or unsubstituted alkynyl, substituted or unsubstituted aryl and substituted or unsubstituted heterocyclyl;
W is selected from the group consisting of -H, substituted or unsubstituted alkyl, substituted or unsubstituted aryl, substituted or unsubstituted aralkyl, substituted or unsubstituted heterocyclyl and substituted or unsubstituted alkenyl;
Ra and Rb are each independently selected from H, OH, OPr, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy, substituted or unsubstituted amino and halogen;
Pr is a hydroxyl protecting group; and
Pm is an amino protecting group;
or its tautomers, salts or solvates thereof.

2. A compound according to claim 1, of formula (Ia) 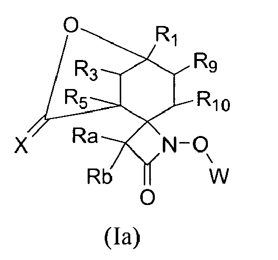 wherein
R₁, R₃, R₉, R₁₀, Ra, Rb, W and X are as defined in claim 1; and
R₅ is selected from the group consisting of hydrogen, -OH or OPr;
or its tautomers, salts or solvates thereof.

3. A compound according to claim 1, of formula (Ib) 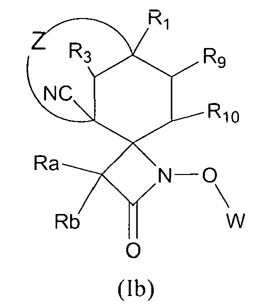 wherein
R₁, R₃, R₉, R₁₀, Ra, Rb, Z and W are as defined in claim 1;
or its tautomers, salts or solvates thereof.

4. A compound according to any of the previous claims, wherein R₁ and R₃ together are -CH₂-O-Pr-O-, wherein Pr is a hydroxyl protecting group.

5. A compound according to any of claims 1, 2 and 3, wherein R₁ and R₃ together are -CH₂-O-C(R₁₁R₁₂)-O-.

6. A compound according to claim 5, wherein R₁₁ is substituted or unsubstituted aryl.

7. A compound according to any of claims 5, 6 and 7, wherein R₁₂ is cyanide.

8. A compound according to any of the previous claims, wherein R₉ and R₁₀ together are -O-Pr-O-, wherein Pr is a hydroxyl protecting group.

9. A compound according to claim 2 of formula (Ia') 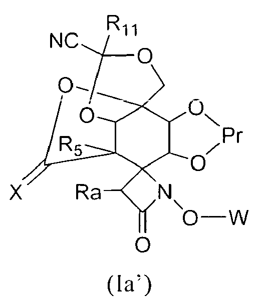
wherein R₅, R₁₁, Ra, X, Pr and W are defined as in claim 2;
or its tautomers, salts or solvates thereof.

10. A compound according to any of claims 1, 2 and 4 to 9, wherein R₅ is hydrogen.

11. A compound according to any of claims 1, 2 and 4 to 9, wherein R₅ is -OH.

12. A compound according to any of claims 1, 2 and 4 to 11, wherein X is O.

13. A compound according to claim 3 of formula (Ib') 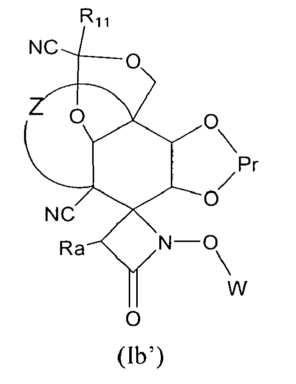 wherein Ra, R₁₁, , Z, Pr and W are defined as in claim 3;
or its tautomers, salts or solvates thereof.

14. A compound according to any of the previous claims, wherein W is aralkyl.

15. A compound according to claim 14, wherein W is -CRcRd-aryl, wherein Rc and Rd are each independently selected from H, OH, OPr, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy, substituted or unsubstituted amino and halogen.

16. A compound according to claim 15, wherein Rc and Rd are different.

17. A compound according to any of claims 15 and 16, wherein W is -CRcH-aryl, wherein Rc is selected from the group consisting of OH, OPr, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted alkoxy, substituted or unsubstituted aryloxy, substituted or unsubstituted amino and halogen.

18. A compound according to claim 15, wherein Rc and Rd are both hydrogen.

19. A compound according to any of claims 1 to 3, wherein R₁ is selected from the group consisting of-CH₂-OC(=O)alkyl and -CH₂-OC(=O)Aryl.

20. A compound according to any of the previous claim, wherein Ra and Rb are different.

21. A compound according to any of claims 1 to 19, wherein Ra and Rb are both hydrogen.

22. Method for the synthesis of a compound of formula (I) which comprises one of the following procedures:
(i) cyclation between the R₂ group and the cyano group of a compound of formula (II) 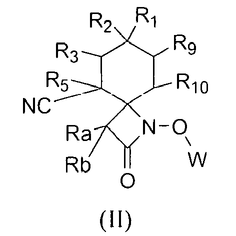 wherein
R₁, R₃, R₉, R₁₀, Ra, Rb and W are as defined in claim 1;
R₅ is selected from the group consisting of hydrogen, -OH and -OPr; and
R₂ is -OH or -OPr, Pr being a hydroxyl protecting group; wherein the cyano group and R₂ are in relative cis positions;
or its tautomers, salts or solvates thereof;
(ii) reacting a compound of formula (III) 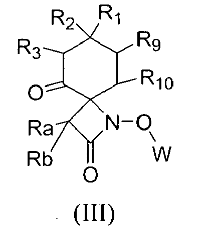 wherein
R₁, R₃, R₉, R₁₀, Ra, Rb and W are as defined in claim 1; and
R₂ is -OH or -OPr, Pr being a hydroxyl protecting group;
or its tautomers, salts or solvates thereof;
in the presence of ⁻CN; and, if necessary, epimerizing the C5 carbon atom of the compound of formula (II) obtained;
(iii) dihydroxylating the double bond between C7 and C10 of a compound of formula (IV) 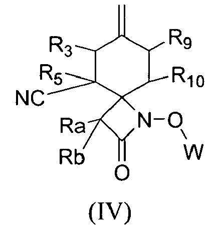 wherein
R₃, R₅, R₉, R₁₀, Ra, Rb and W are as defined in claim 1; and
R₅ is selected from the group consisting of hydrogen, -OH and -OPr;
or its tautomers, salts or solvates thereof;
(iv) hydrogenate a compound of formula (V) 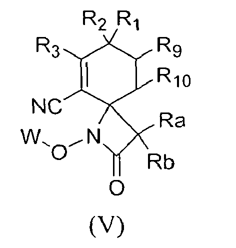 wherein
R₁, R₃, R₉, R₁₀, Ra, Rb and W are as defined in claim 1; and
R₂ is -OH or -OPr;
or its tautomers, salts or solvates thereof; or
(v) diol protection of hydroxyl groups on C5 and C7 of a compound of formula (II) 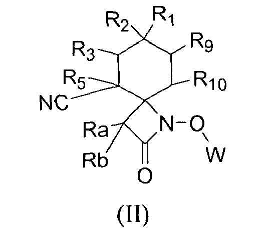 wherein
R₁, R₃, R₉, R₁₀, Ra, Rb and W are as defined in claim 1; and
R₂ and R₅ are both independently a -OH group in cis relative positions;
or its tautomers, salts or solvates thereof.

23. The method according to claim 22, wherein the compound of formula (III) reacts in the presence of TMSCN or Et₂AlCN.

24. The method of any of claims 22 and 23 which comprises
a) reacting a compound of formula (VIII) 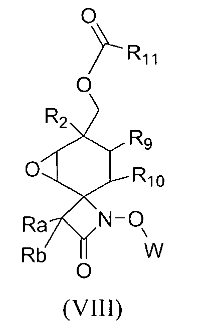
wherein R₂, R₉, R₁₀, R₁₁, Ra, Rb and W are as defined in claim 1, or tautomers, salts or solvates thereof;
in the presence of ⁻CN, to obtain a compound of formula (VII) 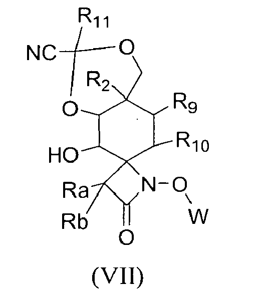
wherein R₂, R₉, R₁₀, R₁₁, Ra, Rb and W are as defined in claim 1, or tautomers, salts or solvates thereof;
b) oxidizing said compound of formula (VII); and
c) reacting the resulting compound of formula (III) in the presence of ⁻CN; and, if necessary, epimerizing the C5 carbon atom of the compound of formula (II)obtained.

25. The method of claim 24, wherein ⁻CN source in steps a) and c) has the same formula.

26. Use of a compound of formula (I) as intermediate in the synthesis of TTX and analogues thereof.
